# EUROPEAN PATENT APPLICATION

(11) **EP 0 922 710 A1**
(43) Date of publication of application: **16.06.1999**
(21) Application number: 98919563.1
(22) Date of filing: 12.05.1998
(51) Int. Cl.: C07K 14/495, A61K 38/17, G01N 33/566

(54) **PEPTIDES PROMOTING THE ACTIVATION OF LATENT TGF-$g(b) AND METHOD FOR SCREENING TGF-$g(b) ACTIVITY REGULATORS**

(30) Priority: 12.05.1997 JP 12068397
(71) Applicant: KYOWA HAKKO KOGYO CO., LTD., Chiyoda-ku, Tokyo 100-8185 (JP)
(72) Inventor: YAMASAKI, Motoo, Tokyo 194-0021 (JP); SHIBATA, Kenji, Tokyo 206-0041 (JP); SATO, Yasufumi, Sendai-shi Miyagi 989-3201 (JP)
(74) Representative: VOSSIUS & PARTNER
(86) International application number: JP9802089
(87) International publication number: WO9851704

(57) **Abstract**

Provided are peptides having an activity to promote the release of active TGF-β from latent TGF-β or an activity to promote the binding of latent TGF-β to a cell membrane which are represented by general formula (I):

R¹-A-R² (I)

(wherein R¹ represents hydrogen, or substituted or unsubstituted alkanoyl, etc.; R² represents hydroxy, or substituted or unsubstituted alkoxy or amino; and A represents an amino acid sequence which is selected from partial sequences of a TGF-β precursor sequence); methods of screening compounds to be used for the treatment or prevention of TGF-β -related diseases which comprise evaluating the above activities; and compounds obtainable by such methods and pharmaceutically acceptable salts thereof.

Said compounds and peptides are useful for the treatment or prevention of diseases such as cancer, diabetic retinopathy, atherosclerosis, etc.

## Description

### Technical Field

The present invention relates to novel peptides which promote conversion of latent TGF-β (TGF-β type ordinarily secreted) into active transforming growth factor-β (hereinafter occasionally abbreviated as active TGF-β or merely as TGF-β) having a variety of physiological activities such as inhibition of cell growth, promotion of cell differentiation, immunosuppression, and stimulation of chemotaxis of fibroblasts and which are useful as therapeutic agents for diseases pointed out to be related to the lack of TGF-β activity and diseases against which administration of exogenous TGF-β is considered to be effective, such as cancer, bone fracture, myocardial infarction, myocardial disorder after ischemia reperfusion, cerebral infarction and retinal detachment.

The present invention also relates to methods of screening compounds which regulate the binding of latent transforming growth factor-β (hereinafter occasionally abbreviated as LTGF-β) to cells or compounds which regulate the release of active TGF-β from latent TGF-β, and to compounds obtainable by the above methods which are useful for the treatment or prevention of TGF-β-related diseases.

### Background Art

In mammals including humans exist some types of TGF-β such as TGF-β1, β2 and β3, and all of them are secreted as inactive LTGF-β [Robert, A.B. & Sporn, M.B., Peptide Growth Factors and Their Roceptors, Handbook of Experimental Pharmacology, Part 1, SPRINGER-VERLAG, Berlin, p. 419-472 (1990)] and need to be activated after the secretion to exhibit their activities. LTGF-β is divided into two types: small molecular weight latent TGF-β (hereinafter abbreviated as SLTGF-β) wherein a latency associated peptide (hereinafter occasionally abbreviated as LAP) is non-covalently bound to TGF-β and large molecular weight latent TGF-β (hereinafter occasionally abbreviated as LLTGF-β) wherein latent TGF-β binding protein (hereinafter occasionally abbreviated as LTBP) is bound to SLTGF-β by SS bond with LAP. LTGF-β is secreted mostly in the form of LLTGF-β [EMBO Journal, 10, 1091 (1991)]. TGF-β and LAP are biosynthesized as the same protein molecule (TGF-β precursor) having a signal peptide and the amino acid sequence thereof is known [Nature, 316, 701 (1985)].

Some protease enzymes have been pointed out to participate in the activation of latent TGF-β, and plasmin has been analyzed most closely among these enzymes. That is, non-covalently bound TGF-β is released by the limited degradation of LAP by plasmin [Journal of Cell Biology, 110, 1361 (1990)]. The analysis of the activation of latent TGF-β by plasmin at the cell level has revealed the following: the activation by plasmin is carried out on the surface of the cell membrane [Journal of Cell Biology, 109, 309 (1989)], binding of latent TGF-β to the cell membrane is necessary for the activation [Journal of Cell Biology, 121, 439 (1993), *ibid.,* 120, 995 (1993), *ibid*., 123, 1249 (1993)], and latent TGF-β is bound to the cell membrane via LAP [Journal of Cell Biology, 123, 1249 (1993), Tohoku Journal of Experimental Medicine, 179, 23 (1996)]. However, it is not clear how the regulation of the binding of latent TGF-β to a cell membrane is associated with the regulation of TGF-β activity.

It is recognized that latent TGF-β is bound to vascular smooth muscle cells, but not to vascular endothelial cells [Journal of Cell Biology, 123, 1249 (1993)].

TGF-β has a variety of physiological activities such as inhibition of cell growth, promotion of cell differentiation, immunosuppression, and stimulation of chemotaxis of fibroblasts. TGF-β is considered to be associated with various diseases. For example, it has been reported that lack of TGF-β activity is related to diabetic retinopathy [Journal of Cell Biology, 109, 309 (1989), Archives of Ophthalmology, 66, 366 (1961)] and initial lesion of atherosclerosis [Nature Medicine, 1, 1067 (1995)]. TGF-β itself is expected to have a therapeutic effect on bone fracture, myocardial infarction, myocardial disorder after ischemia reperfusion, cerebral infarction and retinal detachment [Journal of Cell Biology, 119, 1017 (1992)]. Further, TGF-β is known to inhibit the growth of various cancer cells [Endocrinology, 128, 1981 (1991), Journal of Clinical Investigation, 87, 277 (1991), Cell Growth & Differentiation, 1, 549 (1990)] and is expected as an anti-tumor agent [Proceedings of the National Academy of Science U.S.A., 92, 4254 (1995)].

Only a part of latent TGF-β produced and secreted in vivo is activated and exhibits its activity, and accordingly, it is considered that the activity of TGF-β can be enhanced by increasing the activation efficiency of latent TGF-β in vivo. Therefore, a compound which promotes the activation of latent TGF-β is expected to be effective as a therapeutic agent for diseases pointed out to be related to the lack of TGF-β activity and diseases against which administration of exogenous TGF-β is considered to be effective, for example, cancer, diabetic retinopathy, atherosclerosis, bone fracture, myocardial infarction, myocardial disorder after ischemia reperfusion, cerebral infarction and retinal detachment.

On the other hand, there have been known various diseases basically accompanied by development of extracellular matrix which are caused by advance of TGF-β activation. A substance which inhibits the TGF-β activation is expected to be effective as a therapeutic agent for diseases such as glomerulonephritis, diabetic nephropathy, renal graft rejection, HIV nephropathy, sudden pulmonary fibrosis, autoimmune pulmonary fibrosis, hepatic cirrhosis, venous constrictive hepatopathy (often occurring after treatments of cancer), systemic sclerosis, keloid, eosinophilia-muscle ache syndrome, re-stricture after angioplasty, intraocular fibrosis, rheumatic arthritis and fibrosis such as nasal polyp [Border W.A. & Noble N.A., Transforming growth factor-β in tissue fibrosis, New Engl. J. Med., 331, 1286 (1994) and Border W.A. & Rouslahti E., Transforming growth factor-β in disease: The dark side of tissue repair, J. Clin. Invest., 90, 1, (1992)].

### Disclosure of the Invention

The present invention provides a peptide having an activity to promote the release of active TGF-β from latent TGF-β or an activity to promote the binding of latent TGF-β to a cell membrane, or a pharmaceutically acceptable salt thereof. In one embodiment, the present invention provides a peptide having an activity to promote the release of active TGF-β from latent TGF-β or an activity to promote the binding of latent TGF-β to a cell membrane which is represented by general formula (I):

R¹-A-R² (I)

(wherein R¹ represents hydrogen, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; R² represents hydroxy, substituted or unsubstituted alkoxy, or substituted or unsubstituted amino; and A represents an amino acid sequence which is selected from partial sequences of a TGF-β precursor sequence and in which 1 to 5 amino acid residues may be deleted, substituted or added; and at two amino acid residues selected from the amino acid residues including the N-terminal and C-terminal amino acid residues in the sequence, the N-terminal amino group or a side-chain amino group and the C-terminal carboxyl group or a side-chain carboxyl group may form an amide bond represented by CO-NH or a reversed amide bond represented by NH-CO, or side-chain thiol groups may form a disulfide bond), or a pharmaceutically acceptable salt thereof.

In another embodiment, the present invention provides a peptide having an activity to promote the release of active TGF-β from latent TGF-β or an activity to promote the binding of latent TGF-β to a cell membrane which is represented by general formula (I), wherein A is an amino acid sequence selected from partial sequences of an amino acid sequence selected from the sequences of amino acids 30 to 60, 142 to 186, and 269 to 297 in the human TGF-β1 precursor sequence and the sequences of TGF-β precursors other than human TGF-β1 corresponding to the sequences of amino acids 30 to 60, 142 to 186, and 269 to 297 in the human TGF-β1 precursor sequence when aligned with the human TGF-β1 sequence, and 1 to 5 amino acid residues in said partial sequence may be deleted, substituted or added, or a pharmaceutically acceptable salt thereof.

In another embodiment, the present invention provides a peptide having an activity to promote the release of active TGF-β from latent TGF-β or an activity to promote the binding of latent TGF-β to a cell membrane which is represented by general formula (I), wherein A is any one of the amino acid sequences of SEQ ID NOS: 1-16 in which 1 to 5 amino acid residues may be deleted, substituted or added, or a pharmaceutically acceptable salt thereof.

In another embodiment, the present invention provides a method of screening a compound to be used for the treatment or prevention of TGF-β-related diseases which comprises measuring the amount of latent TGF-β bound to animal cells after addition of latent TGF-β to said cells, measuring the amount of latent TGF-β bound to animal cells after addition of latent TGF-β and a compound to be evaluated to said cells, and evaluating the inhibiting activity or promoting activity of said compound on the binding of latent TGF-β to animal cells from the change in the amount of latent TGF-β bound to animal cells caused by the addition of said compound.

In another embodiment, the present invention provides a method of screening a compound to be used for the treatment or prevention of TGF-β-related diseases which comprises measuring the amount of TGF-β after addition of a peptide represented by general formula (I) or a pharmaceutically acceptable salt thereof to animal cells, measuring the amount of TGF-β after addition of a compound to be evaluated and a peptide represented by general formula (I) or a pharmaceutically acceptable salt thereof to animal cells, and evaluating the inhibiting activity or promoting activity of said compound on the conversion of latent TGF-β into TGF-β from the change in the amount of TGF-β caused by the addition of said compound.

In another embodiment of the present invention, a compound having inhibiting activity or promoting activity on the binding of latent TGF-β to cells or on the conversion of latent TGF-β into TGF-β is obtainable according to either of the above two methods, and a compound to be used for the treatment or prevention of TGF-β-related diseases or a pharmaceutically acceptable salt thereof is provided.

The peptides represented by general formula (I) are hereinafter referred to as Compounds (I).

In the definitions of the groups in general formula (I), the alkanoyl includes alkanoyl groups having 1 to 20 carbon atoms, such as formyl, acetyl, propionyl, butyryl, isobutyryl, valeryl, isovaleryl, pivaloyl, hexanoyl, heptanoyl, lauroyl and icosanoyl.

Exampless of the aryl moiety of the aroyl and the aryloxycarbonyl are phenyl and naphthyl.

Examples of the heteroaryl moiety of the heteroarylcarbonyl and the heteroaryloxycarbonyl are furyl, thienyl, pyridyl, pyrrolyl, pyrazolyl, pyrimidinyl, pyrazinyl, indolyl, quinolyl, isoquinolyl and quinazolinyl.

The alkyl moiety of the alkoxycarbonyl and the alkoxy includes alkyl groups having 1 to 20 carbon atoms, such as methyl, ethyl, propyl, isopropyl, butyl, pentyl, hexyl, heptyl, docyl, dodecyl and icosyl.

The substituted alkanoyl, the substituted alkoxycarbonyl and the substituted alkoxy each has 1 to 3 substituents which are the same or different. Examples of the substituents are hydroxy, carboxyl, alicyclic alkyl groups having 3 to 8 carbon atoms (e.g. cyclopropyl, cyclobutyl, cyclopentyl, cyclohexyl, cycloheptyl and cyclooctyl), substituted or unsubstituted phenyl, and fluorenyl. The substituted phenyl has 1 to 3 substituents which are the same or different. Examples of the substituents are alkyl, alkoxy, hydroxy, nitro, sulfo, cyano and halogen. The halogen includes fluorine, chlorine, bromine and iodine. The alkyl moiety of the alkyl and the alkoxy as the substituents of the substituted phenyl has the same significance as the above-mentioned alkyl moiety of the alkoxycarbonyl and the alkoxy.

The substituted aroyl, the substituted aryloxycarbonyl, the substituted heteroarylcarbonyl and the substituted heteroaryloxycarbonyl each has 1 to 3 substituents which are the same or different. The substituents are the same as the substituents of the above substituted phenyl.

The substituted amino has 1 to 2 substituents which are the same or different, and examples of the substituents are substituted or unsubstituted alkyl and substituted or unsubstituted aryl. The alkyl has the same significance as the above-mentioned alkyl moiety of the alkoxy, etc., including the substituents thereof. The aryl group has the same significance as the above-mentioned aryl moiety of the aroyl and the aryloxycarbonyl, including the substituents thereof.

As the TGF-β precursor sequence, any kind of TGF-β sequence derived from any animal may be employed. Suitable examples are human TGF-β1 (J05114) [Nature, 316, 701 (1985)] (SEQ ID NO: 17), human TGF-β2 (Y00083) [EMBO. J., 6, 3673 (1987)] (SEQ ID NO: 18), human TGF-β3 (J03241) [Proc. Natl. Acad. Sci., USA, 85, 4715 (1988)] (SEQ ID NO: 19), murine TGF-β1 (M13177) [J. Biol. Chem., 261, 4377 (1986)] (SEQ ID NO: 20), murine TGF-β2 (X57413) [Mol. Endocrinol., 3, 1108 (1989)] (SEQ ID NO: 21), murine TGF-β3 (M32745) [Mol. Endocrinol, 3, 1926 (1989)] (SEQ ID NO: 22), rat TGF-β1 (X52498) [Nucleic Acids Res., 18, 3059 (1990)] (SEQ ID NO: 23), rat TGF-β3 (U03491) [J. Biol. Chem., 270, 2722 (1995)] (SEQ ID NO: 24), bovine TGF-β1 (M36271) [Mol. Endocrinol., 1, 693 (1987)] (SEQ ID NO: 25), porcine TGF-β1 (Y00111) [Nucleic Acids Res., 15, 3187 (1987)] (SEQ ID NO: 26), porcine TGF-β3 (X14150) [EMBO J., 7, 3737 (1988)] (SEQ ID NO: 27), canine TGF-β1 (L34956) [Gene, 155, 307 (1995)] (SEQ ID NO: 28), ovine TGF-β1 (X76916) [Gene, 150, 371 (1994)] (SEQ ID NO: 29), chicken TGF-β2 (X58071) [Mol. Endocrinol., 7, 175 (1991)] (SEQ ID NO: 30), chicken TGF-β3 (M31154) [Mol. Endocrinol., 2, 747 (1988)] (SEQ ID NO: 31), chicken TGF-β4 (M31160) [Mol. Endocrinol., 6, 989 (1992)] (SEQ ID NO: 32), simian (African green monkey) TGF-β (M16658) [DNA, 6, 239 (1987)] (SEQ ID NO: 33) and frog (Xenopus laevis) TGF-β5 (J05180) [J. Biol. Chem., 265, 1089 (1990)] (SEQ ID NO: 34). The numbers in parentheses following the names of TGF-β precursor sequences indicate the accession numbers of GenBank.

There is no restriction in employing A as long as A is an amino aid sequence which is selected from partial sequences of a TGF-β precursor sequence in which 1 to 5 amino acid residues may be deleted, substituted or added. It is preferable that A is an amino acid sequence which is a partial sequence of a sequence selected from the sequences of amino acids 30 to 60, 142 to 186, and 269 to 297 in the human TGF-β1 precursor sequence and the sequences of TGF-β precursors other than human TGF-β1 corresponding to the sequences of amino acids 30 to 60, 142 to 186, and 269 to 297 in the human TGF-β1 precursor sequence when aligned with the human TGF-β1 sequence, and in which 1 to 5 amino acid residues may be deleted, substituted or added.

It is particularly preferable that A is a partial sequence of a sequence selected from the sequences of amino acids 30 to 60, 142 to 186, and 269 to 297 in the human TGF-β1 precursor sequence and the sequences of TGF-β precursors other than human TGF-β1 corresponding to the sequences of amino acids 30 to 60, 142 to 186, and 269 to 297 in the human TGF-β1 precursor sequence when aligned with the human TGF-β1 sequence.

The above animal-derived TGF-β precursors which were aligned with the human TGF-β1 precursor sequence are shown in Tables 1-1 to 1-8. The figures before and after each sequence indicate the position numbers of amino acids, and "-" in the amino acid sequences indicates gap positions.

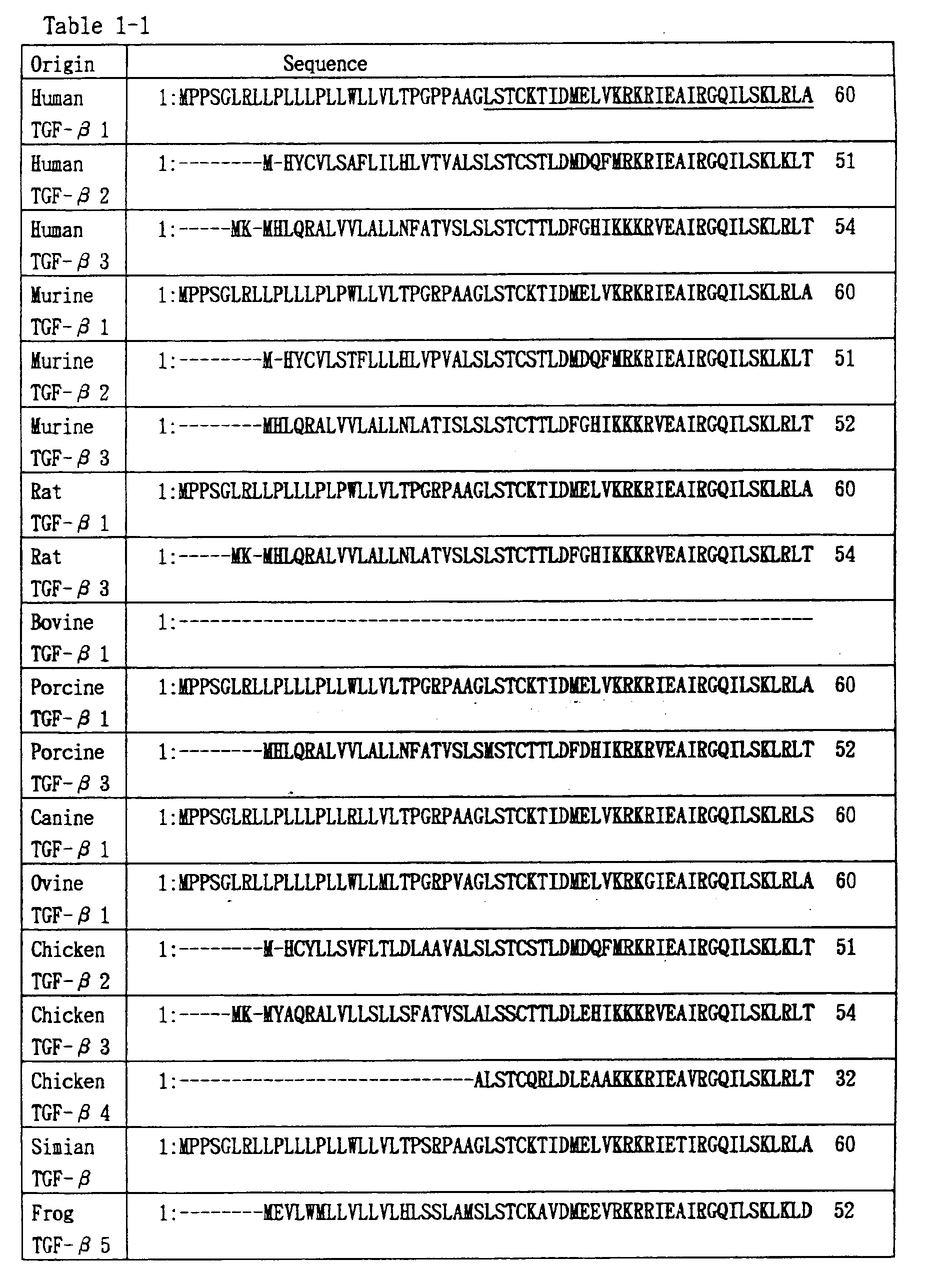

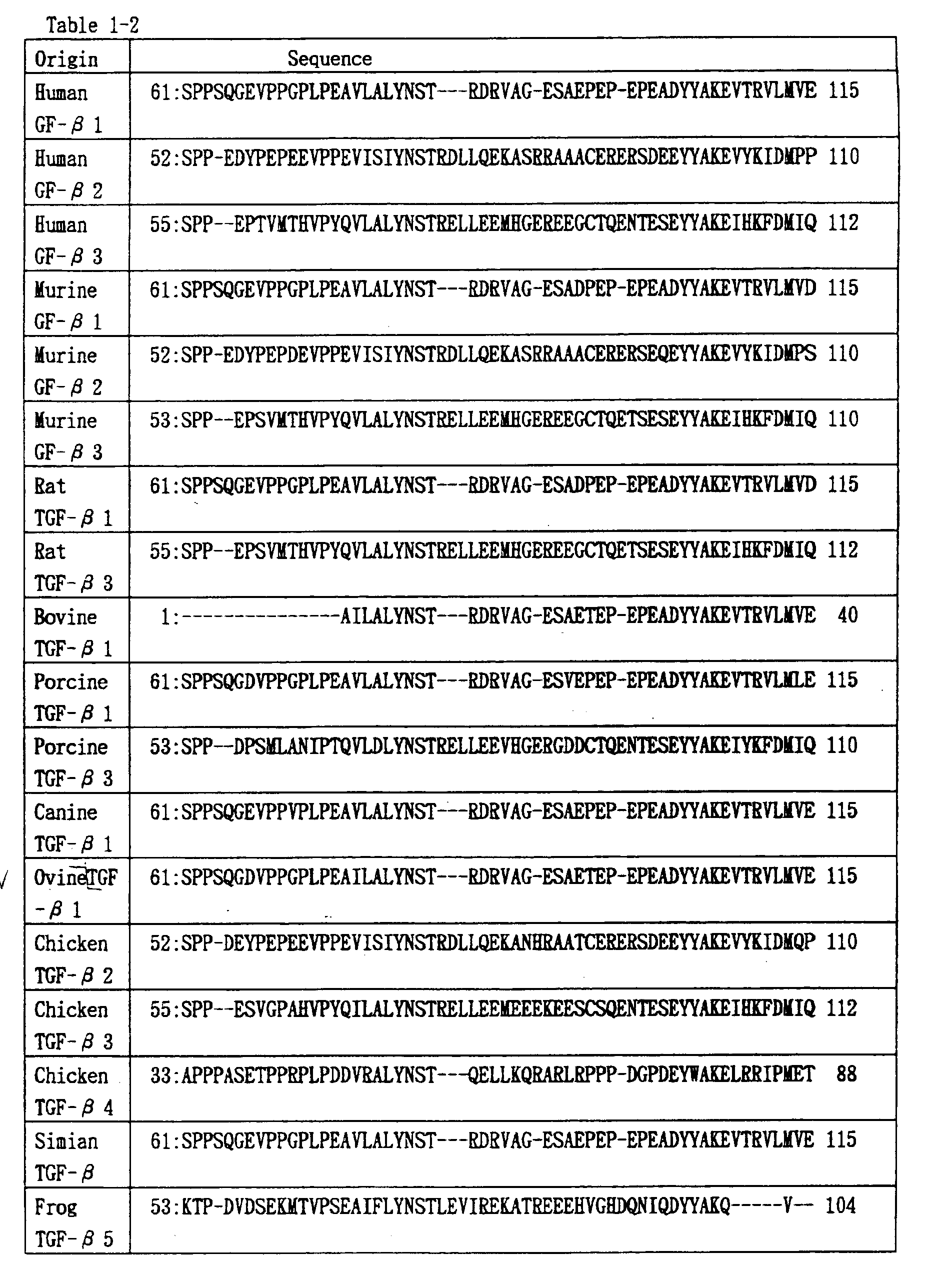

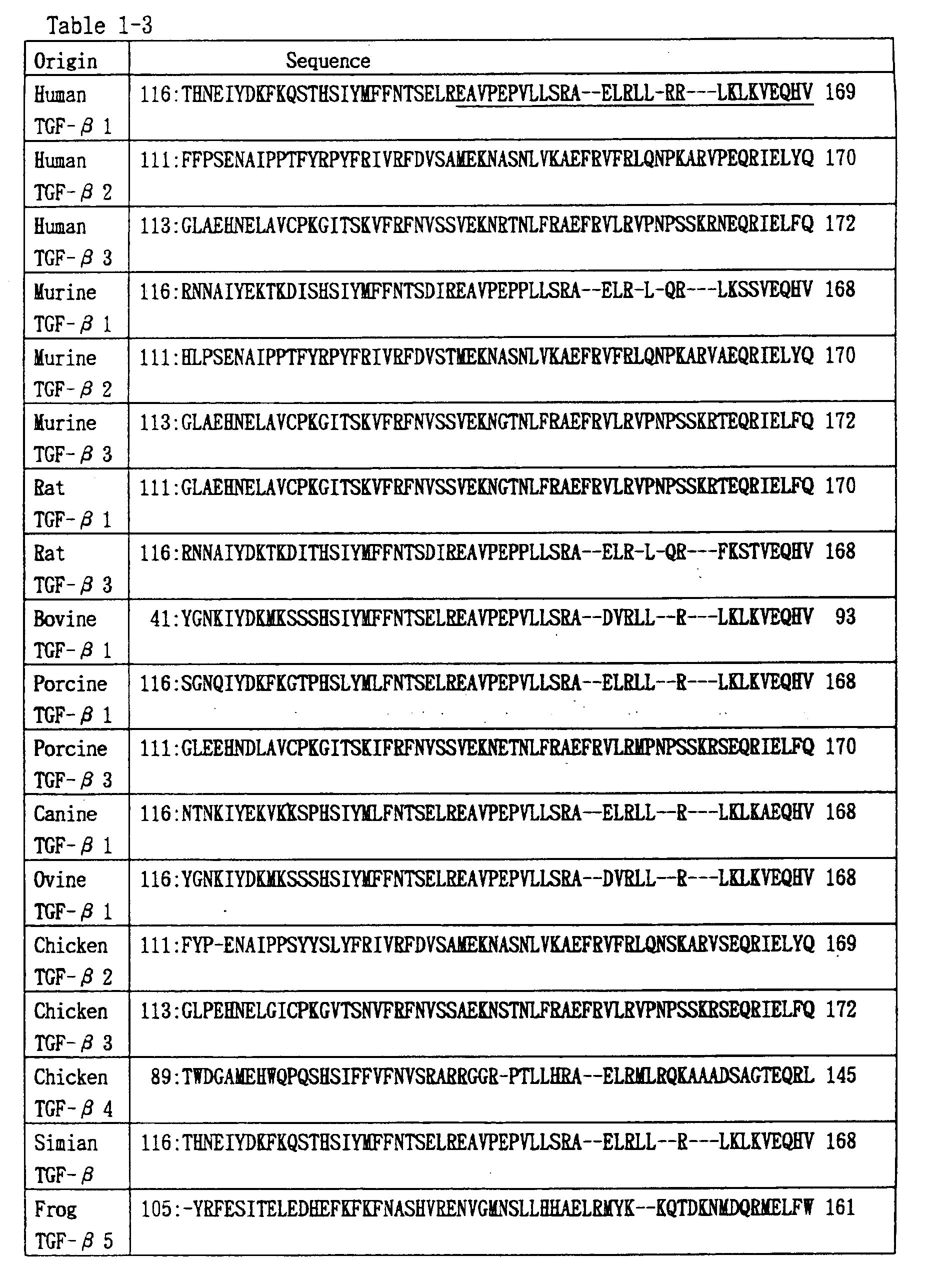

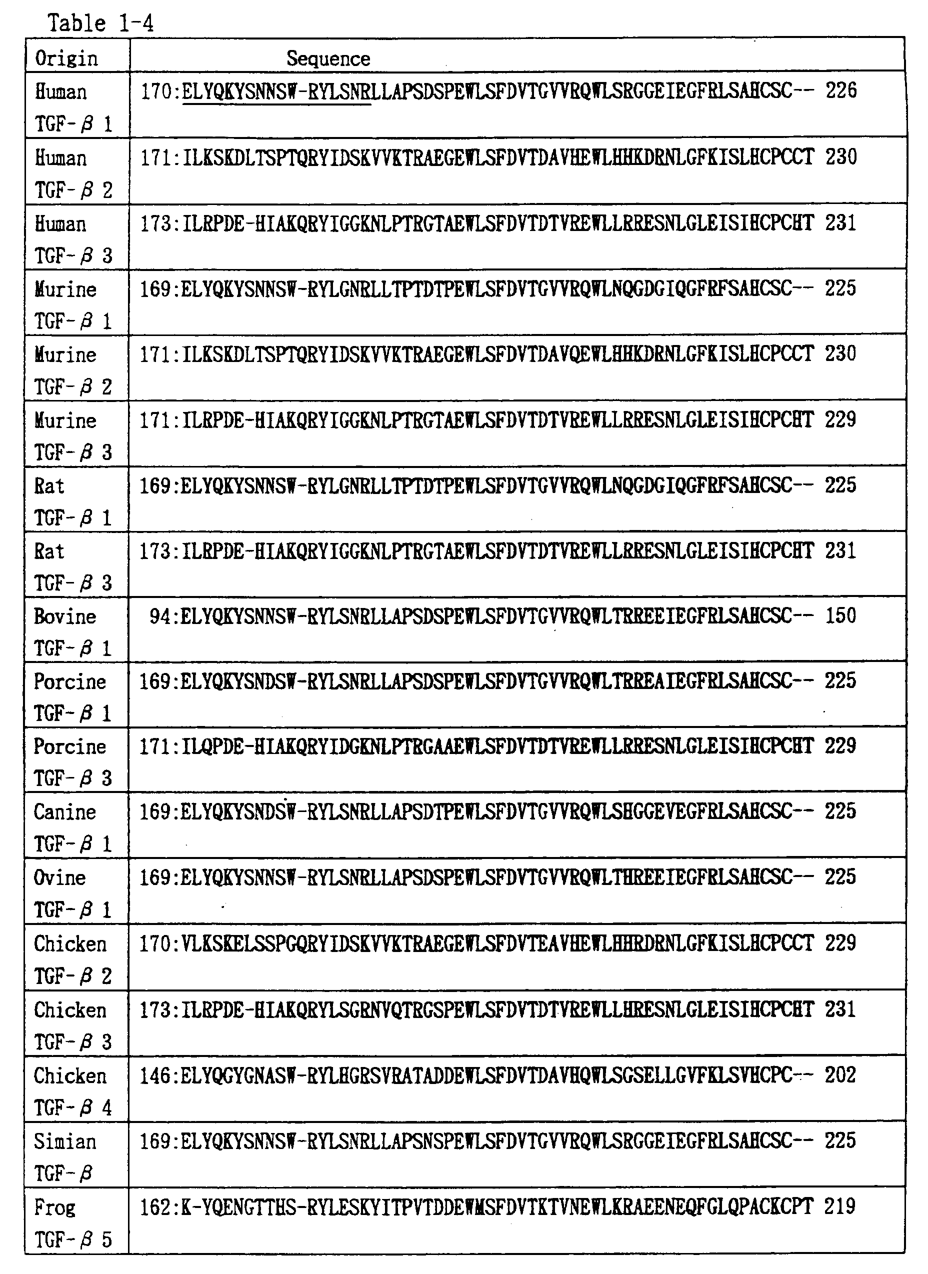

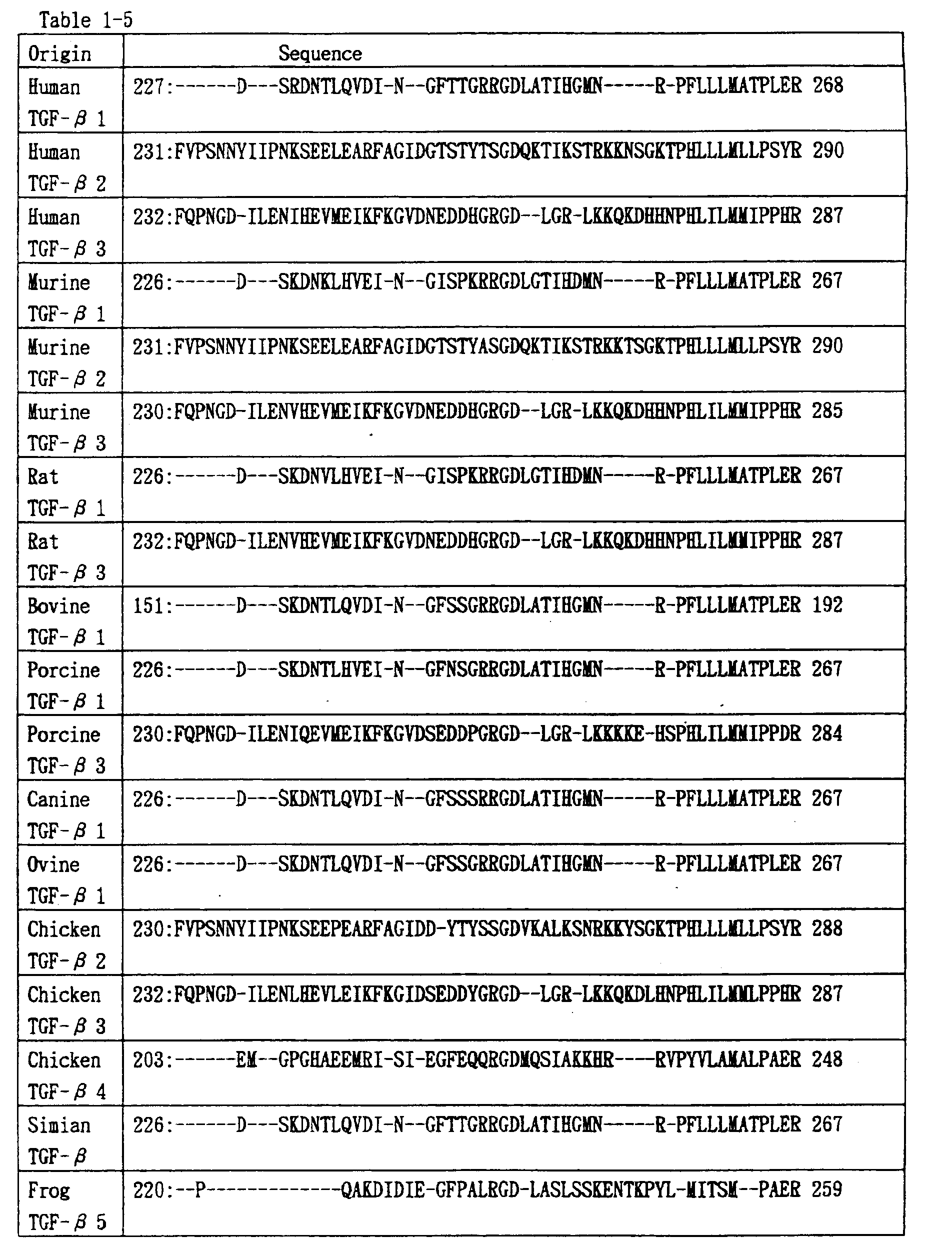

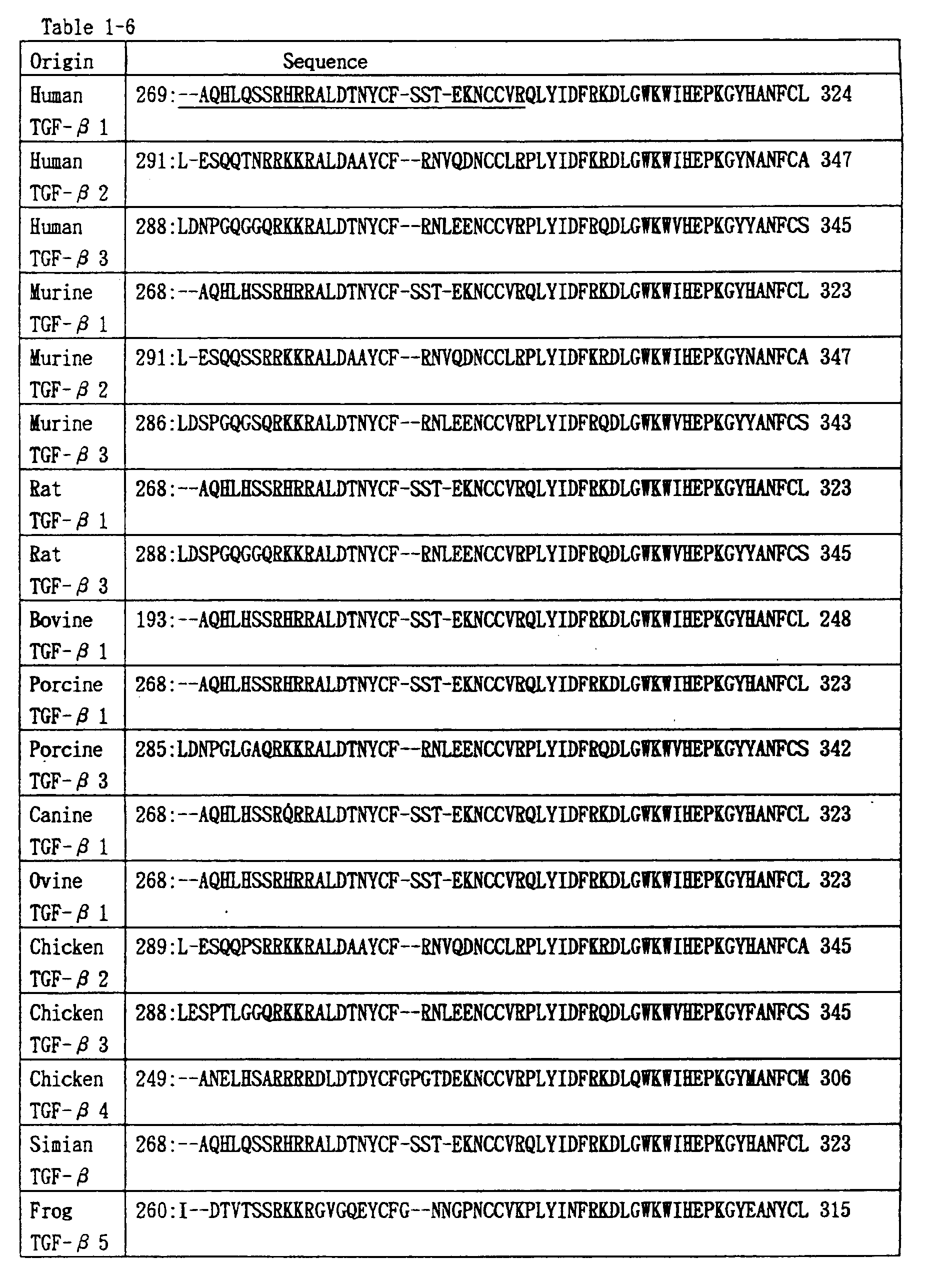

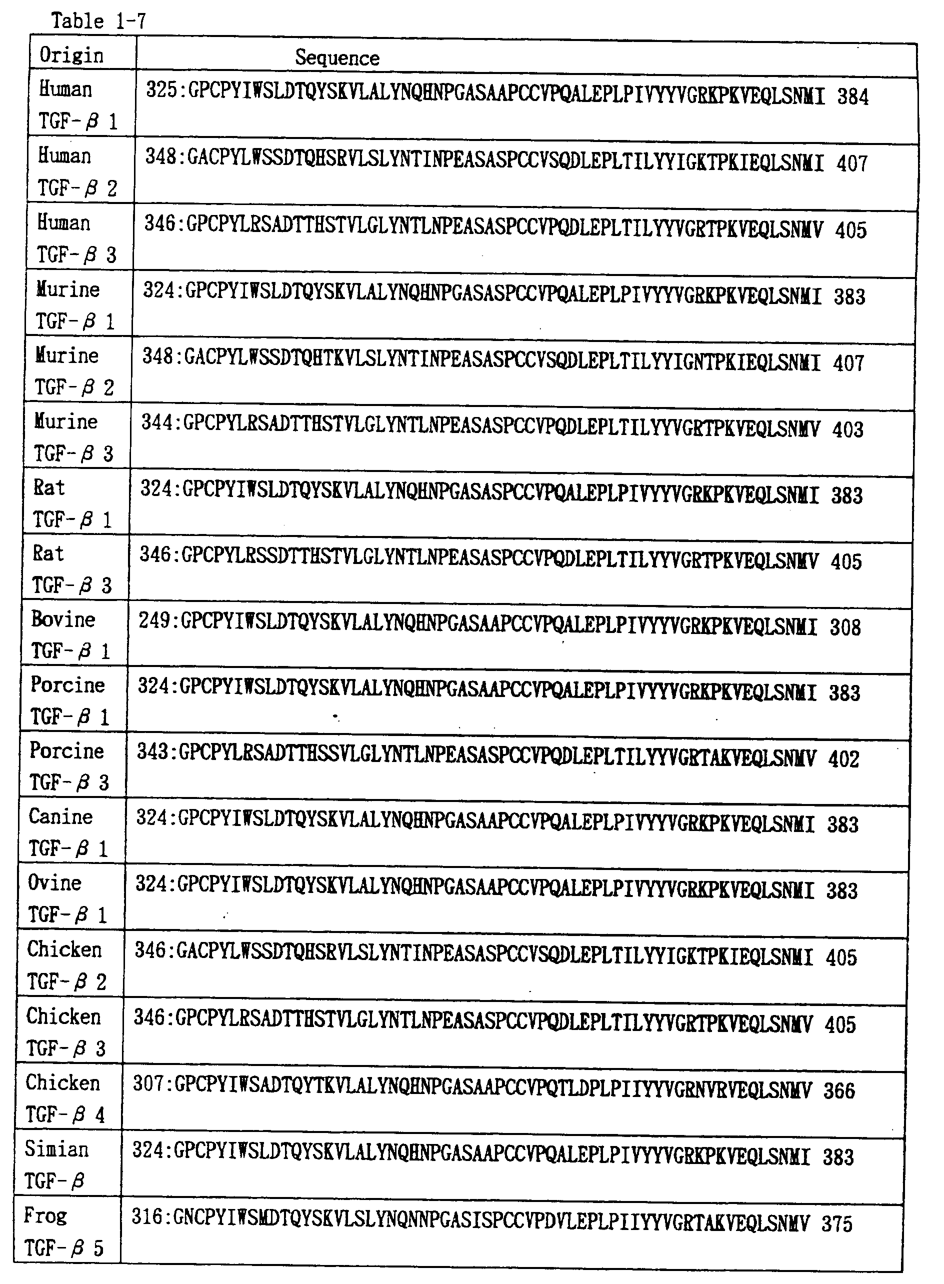

**Table 1-8**

| Origin | Sequence | |
|---|---|---|
| Human TGF-β1 | 385:VRSCKCS | 391 |
| Human TGF-β2 | 408:VKSCKCS | 414 |
| Human TGF-β3 | 406:VKSCKCS | 412 |
| Murine TGF-β1 | 384:VRSCKCS | 390 |
| Murine TGF-β2 | 408:VKSCKCS | 414 |
| Murine TGF-β3 | 404:VKSCKCS | 410 |
| Rat TGF-β1 | 384:VRSCKCS | 390 |
| Rat TGF-β3 | 406:VKSCKCS | 412 |
| Bovine TGF-β1 | 309:VRSCKCS | 315 |
| Porcine TGF-β1 | 384:VRSCKCS | 390 |
| Porcine TGF-β3 | 403:VKSCKCS | 409 |
| Canine TGF-β1 | 384:VRSCKCS | 390 |
| Ovine TGF-β1 | 384:VRSCKCS | 390 |
| Chicken TGF-β2 | 406:VKSCKCS | 412 |
| Chicken TGF-β3 | 406:VKSCKCS | 412 |
| Chicken TGF-β4 | 367:VRACKCS | 373 |
| Simian TGF-β | 384:VRSCKCS | 390 |
| Frog TGF-β5 | 376:VRSCNCS | 382 |

The parts corresponding to the sequences of amino acids 30 to 60, 142 to 186, and 269 to 297 in the human TGF-β1 precursor sequence (the underlined amino acid sequences in the human TGF-β1 precursor sequence in Table 1) are, for example, the sequences of amino acids 21 to 51, 137 to 188, and 291 to 320, respectively, in the human TGF-β2 precursor sequence, and the sequences of amino acids 24 to 54, 139 to 190, and 288 to 318, respectively, in the human TGF-β3 precursor sequence. This kind of alignment can be carried out by the method of Barton & Sternberg [Journal of Molecular Biology, 198, 327 (1987)].

Preferred Compounds (I) are peptides wherein A is an amino acid sequence selected from the sequences of SEQ ID NOS: 1 to 16 in which 1 to 5 amino acid residues may be deleted, substituted or added, and pharmaceutically acceptable salts thereof. Particularly preferred are peptides wherein A is an amino acid sequence selected from the sequences of SEQ ID NOS: 1 to 16.

The expression "1 to 5 amino acid residues may be deleted, substituted or added in the sequence" herein means that the sequence may contain deletion, substitution or addition of a single or plural amino acid residues at a single or plural arbitrarily selected positions therein, and the total number of such residues deleted, substituted or added is 1 to 5, which deletion, substitution and addition may be simultaneously contained in the sequence. It does not matter whether or not the substituted or added amino acid is a natural one.

Examples of the addition are addition of amino acids having a thiol group (e.g. cysteine and homocysteine) or organic groups at both ends of the sequence. Examples of the substitution are substitution of a cysteine residue existing in the sequence to a serine or alanine residue, and substitution of a serine or alanine residue to a cysteine residue. A disulfide bond may be formed between two thiol groups contained in the sequence for cyclization. An amide bond represented by CO-NH or a reversed amide bond represented by NH-CO may be formed between the N-terminal amino group or a side-chain amino group and the C-terminal carboxyl group or a side-chain carboxyl group for cyclization.

Examples of the natural amino acids are glycine, L-alanine, L-threonine, L-aspartic acid, L-asparagine, L-glutamic acid, L-glutamine, L-valine, L-leucine, L-serine, L-methionine, L-isoleucine, L-phenylalanine, L-tyrosine, L-lysine, L-arginine, L-histidine, L-proline, L-cysteine and L-tryptophan.

The term latent TGF-β includes small molecular weight latent TGF-β (SLTGF-β) comprising TGF-β and LAP which inhibits the activity thereof, and large molecular weight latent TGF-β (LLTGF-β) comprising TGF-β, LAP which inhibits the activity

thereof, and LTBP. The pharmaceutically acceptable salts of the compounds obtainable by the method of the present invention and Compounds (I) include acid addition salts, metal salts, organic base addition salts, etc. Examples of the pharmaceutically acceptable acid addition salts are inorganic acid addition salt such as hydrochloride, sulfate and phosphate, and organic acid addition salts such as acetate, maleate, fumarate, tartrate and citrate. Examples of the pharmaceutically acceptable metal salts are alkali metal salts such as sodium salt and potassium salt, alkaline earth metal salts such as magnesium salt and calcium salt, aluminum salt and zinc salt. Examples of the pharmaceutically acceptable organic base addition salts are salts with primary amines, e.g. methylamine, ethylamine and aniline, secondary amines, e.g. dimethylamine, diethylamine, pyrrolidine, piperidine, morpholine and piperazine, and tertiary amines, e.g. trimethylamine, triethylamine, N,N-dimethylaniline and pyridine, and ammonium salt.

The abbreviations for amino acids and their protecting groups used herein are described below.

The abbreviations for amino acids and their protecting groups follow the recommendations by IUPAC-IUB Joint Commission on Biochemical Nomenclature [European Journal of Biochemistry, 138, 9 (1984)].

The abbreviations for amino acids and their protecting groups are as follows, unless otherwise specified.
- Gly or G;: Glycine
- Ala or A;: L-Alanine
- Thr or T;: L-Threonine
- Asp or D;: L-Aspartic acid
- Asn or N;: L-Asparagine
- Asx;: L-Aspartic acid or L-asparagine
- Glu or E;: L-Glutamic acid
- Gln or Q;: L-Glutamine
- Glx;: L-Glutamic acid or L-glutamine
- Val or V;: L-Valine
- Leu or L;: L-Leucine
- Ser or S;: L-Serine
- Met or M;: L-Methionine
- Ile or I;: L-Isoleucine
- Phe or F;: L-Phenylalanine
- Tyr or Y;: L-Tyrosine
- Lys or K;: L-Lysine
- Arg or R;: L-Arginine
- His or H;: L-Histidine
- Pro or P;: L-Proline
- Cys or C;: L-Cysteine
- Trp or W;: L-Tryptophan
- Fmoc;: 9-Fluorenylmethyloxycarbonyl
- t-Bu;: t-Butyl
- Trt;: Trityl
- Pmc;: 2,2,5,7,8-Pentamethylchroman-6-sulfonyl
- Boc;: t-Butyloxycarbonyl

The abbreviations for side-chain-protected amino acids are as follows.
- Fmoc-Asp(Ot-Bu)-OH;: N^{α}-9-Fluorenylmethyloxycarbonyl-L-aspartic acid β-t-butyl ester
- Fmoc-Glu(Ot-Bu)-OH;: N^{α}-9-Fluorenylmethyloxycarbonyl-L-glutamic acid γ-t-butyl ester
- Fmoc-Thr(t-Bu)-OH;: N^{α}-9-Fluorenylmethyloxycarbonyl-O-t-butyl-L-threonine
- Fmoc-Ser(t-Bu)-OH;: N^{α}-9-Fluorenylmethyloxycarbonyl-O-t-butyl-L-serine
- Fmoc-Tyr(t-Bu)-OH;: N^{α}-9-Fluorenylmethyloxycarbonyl-O-t-butyl-L-tyrosine
- Fmoc-Lys(Boc)-OH;: N^{α}-9-Fluorenylmethyloxycarbonyl-N^{ε}-t-butyloxycarbonyl-L-lysine
- Fmoc-Asn(Trt)-OH;: N^{α}-9-Fluorenylmethyloxycarbonyl-N^{γ}-trityl-L-asparagine
- Fmoc-Gln(Trt)-OH;: N^{**α**}-9-Fluorenylmethyloxycarbonyl-N^{δ}-trityl-L-glutamine
- Fmoc-Arg(Pmc)-OH;: N^{α}-9-Fluorenylmethyloxycarbonyl-N^{g}-2,2,5,7,8-pentamethylchroman-6-sulfonyl-L-arginine
- Fmoc-His(Trt)-OH;: N^{α}-9-Fluorenylmethyloxycarbonyl-N^{im}-trityl-L-glutamine
- Fmoc-Cys(Trt)-OH;: N^{α}-9-Fluorenylmethyloxycarbonyl-S-trityl-L-cysteine
- Fmoc-Trp(Boc)-OH;: N^{α}-9-Fluorenylmethyloxycarbonyl-N^{ind}-t-butyloxycarbonyl-L-tryptophan

The abbreviations for reaction solvents, reaction reagents, etc. are as follows.
- PyBOP;: Benzotriazol-1-yloxytrispyrrolidinophosphonium hexafluorophosphate
- HOBt;: N-Hydroxybenzotriazole
- DCC;: Dicyclohexylcarbodiimide
- NMM;: N-Methylmorpholine
- DMF:: N,N-Dimethylformamide
- NMP;: N-Methylpyrrolidone
- TFA;: Trifluoroacetic acid
- DTT;: Dithiothreitol
- HBTU;: 2-(1H-Benzotriazol-1-yl)-1,1,3,3-tetramethyluronium hexafluorophosphate
- DIPC;: N,N'-Diisopropylcarbodiimide
- DIEA;: N,N-Diisopropylethylamine
- DCM;: Dichloromethane

The processes for producing Compounds (I) are described below.

Compounds (I) can be synthesized by general liquid phase or solid phase peptide synthetic methods [Fundamentals and Experiments of Peptide Synthesis, Nobuo Izumiya, et al., Maruzen (1985)], or appropriate combinations thereof. Compounds (I) can also be synthesized by using an automatic peptide synthesizer. That is, the peptide synthesis can be carried out by the use of commercially available peptide synthesizers from Shimadzu Corporation, Applied Biosystems Inc., U.S.A. (ABI), Advanced ChemTech Inc., U.S.A. (ACT), etc. using an appropriately side-chain-protected N^{α}-9-fluorenylmethyloxycarbonyl amino acid or N^{α}-t-butyloxycarbonyl amino acid according to respective synthesis programs.

The protected amino acids which are starting materials for the synthesis of Compounds (I) and carrier resins are available from ABI, Shimadzu Corporation, Kokusan Chemical Works Co., Ltd., Nova Biochem Co., Watanabe Chemical Industries, Ltd., ACT, and Peptide Institute Inc.

Cyclization may be carried out after all the constituent amino acid residues and organic groups are bonded by a liquid phase method, a solid phase method or a combination thereof, or in the course of elongation of the peptide chain. In the latter case, the obtained cyclization product is subjected to further condensation with amino acid residues or organic groups to prepare Compound (I). The cyclic structure may be formed by forming, at the final step of the process, a disulfide bond, an amide bond or a reversed amide bond which forms a cyclic structure in general formula (I), or by forming an amide bond in an ordinary sequence, after the above bonds are formed, between an amino acid residue and the adjacent amino acid residue which are to be constituents or the cyclic structure. The cyclization process is described in detail below.

### 1. Cyclization by disulfide bond formation

First a peptide which has, at two positions in the sequence, amino acid residues having appropriately protected thiol groups is prepared by a solid phase method, a liquid phase method or a combination thereof. Then, protecting groups other than the thiol-protecting groups are removed, followed by removal of the thiol-protecting groups. The thus obtained precursor peptide is subjected to oxidation reaction and the product is purified by general purification steps in organic chemical reactions to give the desired peptide having a cyclic structure with a disulfide bond.

### 1-1 Cyclization by disulfide bond formation according to a liquid phase method

The peptide having a cyclic structure with a disulfide bond can be prepared by subjecting the above precursor peptide to air oxidation or reaction with an oxidizing agent in an inert solvent. The reaction is carried out at a peptide concentration of 0.5-5000 µmol/l, preferably 50-500 µmol/l. As the solvent, buffers such as 50 mM - 1 M tris(hydroxymethyl)aminomethane-hydrochloric acid (Tris-HCl) buffer adjusted to pH 4-9, preferably pH 6-8, 5-50% aqueous acid, water, and organic solvents such as DMF, DMSO, acetonitrile, tetrahydrofuran, methanol and ethanol can be used alone or in combination. Examples of the oxidizing agents are potassium ferricyanide and iodine, which are used respectively in the amounts of 0.1-1 time and 0.5-5 times (preferably one time) that of the precursor peptide (weight:weight). DMSO can also be used as the oxidizing agent at a concentration of 10-50%. The reaction is usually carried out at 0-40°C for one hour to one week. In some cases, the yield of oxidation product can be increased by addition of glutathione, and the reaction may be carried out in the presence of oxidized glutathione in an amount of 0.5-5 times that of the precursor peptide (weight:weight) and reduced glutathione in an amount of one-half the weight of oxidized glutathione [Journal of American Chemical Society, 103, 5867 (1981); Development of Medicines, second series, vol. 14, Peptide Synthesis, p. 239, compiled under the supervision of Haruaki Yajima, Hirokawa Shoten (1991)]. When iodine is used as the oxidizing agent, zinc powder is added after the completion of reaction until the color of iodine disappears from the reaction mixture, and the mixture is purified as such, or after concentration under reduced pressure, by means of various kinds of chromatography. When potassium ferricyanide is used as the oxidizing agent, the reaction mixture is made weakly acidic by addition of acetic acid and then is purified as such, or after concentration under reduced pressure, by means of various kinds of chromatography. Alternatively, an anion exchange resin such as Dowex 1X2 (AcO-) (Dow Chemical Co.) may be added to the reaction mixture to remove excess potassium ferricyanide (ferricyan ion and ferrocyan ion) by adsorption, and then the mixture is purified as such, or after concentration under reduced pressure, by means of various kinds of chromatography.

It is also possible to pyridylsulfenylate or 2-nitropyridylsulfenylate one of the thiol groups and then force the cyclization reaction to proceed to completion simultaneously with the selective removal of the other thiol group [International Journal of Peptide and Protein Research, 29, 162 (1987)]. The solvent, reaction temperature, reaction time, etc. for the cyclization reaction are substantially the same as described above. Further, after the protection groups of the two thiol groups are removed, an equivalent amount of a reagent for pyridylsulfenylation or 2-nitropyridylsulfenylation may be introduced. Pyridylsulfenylation can be carried out by adding 1-3 equivalents of a reagent such as 2,2'-dithiodipyridine to a solvent containing the peptide, followed by stirring. 2-Nitropyridylsulfenylation can be carried out in a similar manner. The solvent, reaction temperature, reaction time, etc. for the reaction are substantially the same as described above [Peptide Chemistry, 1991, 125 (1992)].

### 1-2 Cyclization by disulfide bond formation according to a solid phase method

A peptide which has, at two positions in the sequence, amino acid residues having appropriately protected thiol groups is elongated by a solid phase method. Before cleavage of the peptide from the resin, the thiol-protecting groups are selectively removed and the peptide is subjected to oxidation reaction to prepare a peptide moiety having a cyclic structure. Then, the peptide is cleaved from the resin and the remaining protecting groups are removed, whereby the desired peptide having a cyclic structure is obtained.

Examples of the thiol-protecting groups include acetamidomethyl (Acm) group and trityl (Trt) group. By reaction of the protected peptide on the resin with iodine in an appropriate solvent such as DMF or DCM, Acm group and Trt group are removed and an intramolecular disulfide bond is formed. The reaction is carried out using 0.5-2 ml of a solvent for 50 mg of the resin, and iodine in an amount of 0.5-5 times, preferably one time the calculated weight of the peptide on the resin. The reaction is usually carried out at 0-40°C for one hour to one week. After the completion of reaction, the resin is subjected to a usual treatment in a solid phase method, that is, washing with a small amount of a solvent such as DMF or DCM, and then subjected to the subsequent reaction.

The pyridylsulfenylation or 2-nitropyridylsulfenylation of one of the thiol groups as described in 1-1 above can be applied to a solid phase method. The solvent, reaction temperature, reaction time, etc. for the cyclization reaction are substantially the same as described above. Further, similarly to the above-described liquid phase method, an equivalent amount of a reagent for pyridylsulfenylation or 2-nitropyridylsulfenylation may be introduced after the protecting groups of the two thiol groups are removed. Pyridylsulfenylation can be carried out by adding 1-3 equivalents of a reagent such as 2,2'-dithiodipyridine to the resin swollen with a solvent, followed by stirring. 2-Nitropyridylsulfenylation can be carried out in a similar manner. The solvent, reaction temperature, reaction time, etc. for the reaction are substantially the same as in the above cyclization reaction in the solid phase method.

### 2. Cyclization by amide bond or reversed amide bond formation

By a solid phase method, a liquid phase method, or a combination thereof, a peptide is prepared which has, at two positions in the sequence, an amino acid residue having an appropriately protected amino group and an amino acid residue having an appropriately protected carboxyl group and in which the side chains, N-terminus and C-terminus are protected. After the amino- and carboxyl-protecting groups are selectively removed, the peptide is subjected to intermolecular condensation, followed by general purification steps in organic chemical reactions to give a peptide which has a cyclic structure and in which the side chains, N-terminus and C-terminus are protected. Then, the remaining protecting groups are removed, whereby the desired peptide is obtained. The desired peptide can also be prepared by first preparing a peptide moiety having a cyclic structure and then elongating it.

### 2-1 Cyclization by amide bond or reversed amide bond formation according to a liquid phase method

By a solid phase method, a peptide is prepared which has, at two positions in the sequence, an amino acid residue having an appropriately protected amino group and an amino acid residue having an appropriately protected carboxyl group. Before cleavage of the peptide from the resin, the amino- and carboxyl-protecting groups are selectively removed, and the obtained peptide having free amino group and free carboxyl group is subjected to condensation reaction to give a peptide moiety having a cyclic structure. Then, the peptide is cleaved from the resin and the remaining side-chain-protecting groups are removed, whereby the desired peptide having a cyclic structure is obtained.

When 4-methyltrityl group is used as the amino-protecting group, it can be removed by reaction using acetic acid/trifluoroethanol/DCM (1/2/7). The reaction is usually carried out at 0-40°C for 0.5-6 hours. After the completion of reaction, the peptide is precipitated by addition of diethyl ether, etc., followed by removal of the solvent, if necessary under reduced pressure. General purification steps in organic chemical reactions including such step are applicable as may be required.

When allyloxycarbonyl group is used as the amino-protecting group and allyl ester group is used as the carboxyl-protecting group, these protecting groups can be simultaneously removed by reaction with a reducing agent in the presence of a palladium catalyst. It is also possible to use only allyl ester group as the carboxyl-protecting group. Any zerovalent palladium catalysts for homogenous system can be used in the reaction. Suitable catalysts include tetrakis(triphenylphosphine)palladium(0) and palladium(II) acetate-triphenylphosphine. The catalyst is used in an amount of 0.01-1 equivalent, preferably 0.1-0.5 equivalent, based on the above protecting groups. Further, additives such as formic acid, formic acid-triethylammonium, tributyltin hydride, triphenyltin hydride, trimethylhydrosilane, sodium borohydride, acetic acid, and acetic acid-NMM are added in an amount of one equivalent to excess based on the above protecting groups. As a solvent, ether, tetrahydrofuran, acetonitrile, DMF, chloroform, etc. are used alone or in combination. For 1 mM of allyloxycarbonyl group and allyl ester group are added the above reagents and 3-10 ml of the solvent. The reaction is carried out at -20 to 80°C, preferably 0 to 30°C for 10 minutes to 6 hours. After the completion of reaction, general purification steps in organic chemical reactions can be applied.

The obtained peptide is then subjected to reaction for forming an intermolecular amide bond between the free amino group and the free carboxyl group. Typical amide bond formation reactions for cyclization are described below. Common reaction conditions are as follows. As a solvent, DMF, NMP, methylene chloride, chloroform, acetonitrile, tetrahydrofuran, etc. are used alone or in combination. The peptide is used at a concentration of 0.5-5000 µmol/l, preferably 50-500 µmol/l. The reaction is carried out usually at 0-40°C, preferably 4-25°C, with stirring for 3 hours to one week. After the completion of reaction, general purification steps in organic chemical reactions can be applied.

Amide bond formation reaction can be carried out by using carbodiimide such as dicyclohexylcarbodiimide (DCC) or water-soluble carbodiimide (WSC) in an amount of 1-10 equivalents based on the carboxyl group. NMM, DIEA or sodium hydrogencarbonate is added in an amount of 1.5-2 equivalents based on carbodiimide. If necessary, HOBt or HONSu may be added in an equimolar amount based on carbodiimide.

Amide bond formation reaction can also be carried out by using diphenylphosphoryl aside (DPPA) or diethyl phosphorocyanidate (DEPC) in an amount of 1-10 equivalents based on the carboxyl group. NMM, DIEA or sodium hydrogencarbonate is added in an amount of 1.5-2 equivalents based on carbodiimide.

Further, amide bond formation reaction can be carried out by using PyBOP or HBTU in an amount of 1-10 equivalents, preferably 2-5 equivalents based on the carboxyl group, and HOBt in an equimolar amount based on PyBOP or HBTU. NMM, DIEA or sodium hydrogencarbonate is added in an amount of 1.5-2 equivalents based on PyBOP or HBTU.

It is also possible to convert the carboxyl group into an active ester, selectively remove the amino-protecting group, and then form an amide bond. Examples of the active esters are p-nitrophenyl ester, pentafluorophenyl ester, and N-oxysuccinimide ester. The active esters can be formed by various methods. For example, DCC is added in an amount of 1-10 equivalents based on the carboxyl group, together with an equimolar amount of p-nitrophenol, pentafluorophenol or HONSu, and the mixture is stirred at 0-5°C for one hour to one day, followed by removal of the formed dicyclohexylurea (DCUrea) by filtration. Then, purification can be carried out by general purification steps in organic chemical reactions. The solvent, reaction temperature, reaction time, etc. for the formation of active esters are substantially the same as in the above amide bond formation reactions.

### 2-2 Cyclization by amide bond or reversed amide bond formation according to a solid phase method

By a solid phase method, a peptide is prepared which has, at two positions in the sequence, an amino acid residue or an organic group having an appropriately protected amino group and an amino acid residue or an organic group having an appropriately protected carboxyl group. Before cleavage of the peptide from the resin, the amino- and carboxyl-protecting groups are selectively removed, and the obtained peptide having free amino group and free carboxyl group is subjected to condensation reaction to give a peptide moiety having a cyclic structure. Then, the peptide is cleaved from the resin and the remaining side-chain-protecting groups are removed, whereby the desired peptide having a cyclic structure is obtained.

When 4-methyltrityl group is used as the amino-protecting group, it can be removed by reaction using 0.5-2 ml of acetic acid/trifluoroethanol/DCM (1/2/7) for 50 mg of the resin. The reaction is usually carried out at 0-40°C for 0.5-6 hours. After the completion of reaction, the resin is subjected to a usual treatment in a solid phase method, that is, washing with a small amount of a solvent such as DMF, and then subjected to the subsequent reaction.

When allyloxycarbonyl group is used as the amino-protecting group and allyl ester group is used as the carboxyl-protecting group, these protecting groups can be removed by reaction using, for example, a chloroform solution containing 0.1-0.2 M tetrakis (triphenylphosphine)palladium(0), 5% acetic acid and 2.5% NMM. For 1 mM of allyloxycarbonyl group and allyl ester group is added 3-10 ml of the above chloroform solution. The reaction is usually carried out at 0-40°C for 0.5-6 hours. After the completion of reaction, the resin is subjected to a usual treatment in a solid phase method, that is, washing with a small amount of a solvent such as DMF, and then subjected to the subsequent reaction.

The subsequent reaction is carried out by using, for 50 mg of the resin, 1 ml of an organic solvent such as DMF, DCM or NMP containing PyBOP or HBTU in an amount of 1-10 equivalents, preferably 2-5 equivalents based on the calculated quantity of the carboxyl group on the resin, HOBt in an equimolar amount based on PyBOP or HBTU, and NMM or DIEA in an amount of 1.5-2 equivalents based on PyBOP or HBTU. The reaction is carried out usually at 0-40°C, preferably 4-25°C, with stirring for 3 hours to one week. After the completion of reaction, the resin is subjected to a usual treatment in a solid phase method, that is, washing with a small amount of a solvent such as DMF, and then subjected to the subsequent reaction.

Compounds (I) obtained by the above processes can be purified by high performance liquid chromatography (hereinafter referred to as HPLC) using a reversed-phase silica gel columns such as C-4, C-8 and C-18, or column chromatography or thin layer chromatography such as gel filtration using partition resins, adsorption resins, ion-exchange resins, silica gel, chemically-modified silica gel, reversed-phase silica gel, alumina, diatomaceous earth or magnesium silicate.

The pharmaceutically acceptable salts of Compound (I) are obtained according to an ordinary method. That is, the acid addition salts and organic base addition salts of Compound (I) are obtained by dissolving Compound (I) in an aqueous solution of the corresponding acid or organic base, followed by freeze-drying. The metal salts of Compound (I) are obtained by dissolving Compound (I) in an aqueous solution containing the corresponding metal ion, followed by purification by gel filtration or HPLC.

Specific examples of Compounds (I) are shown in Table 2.

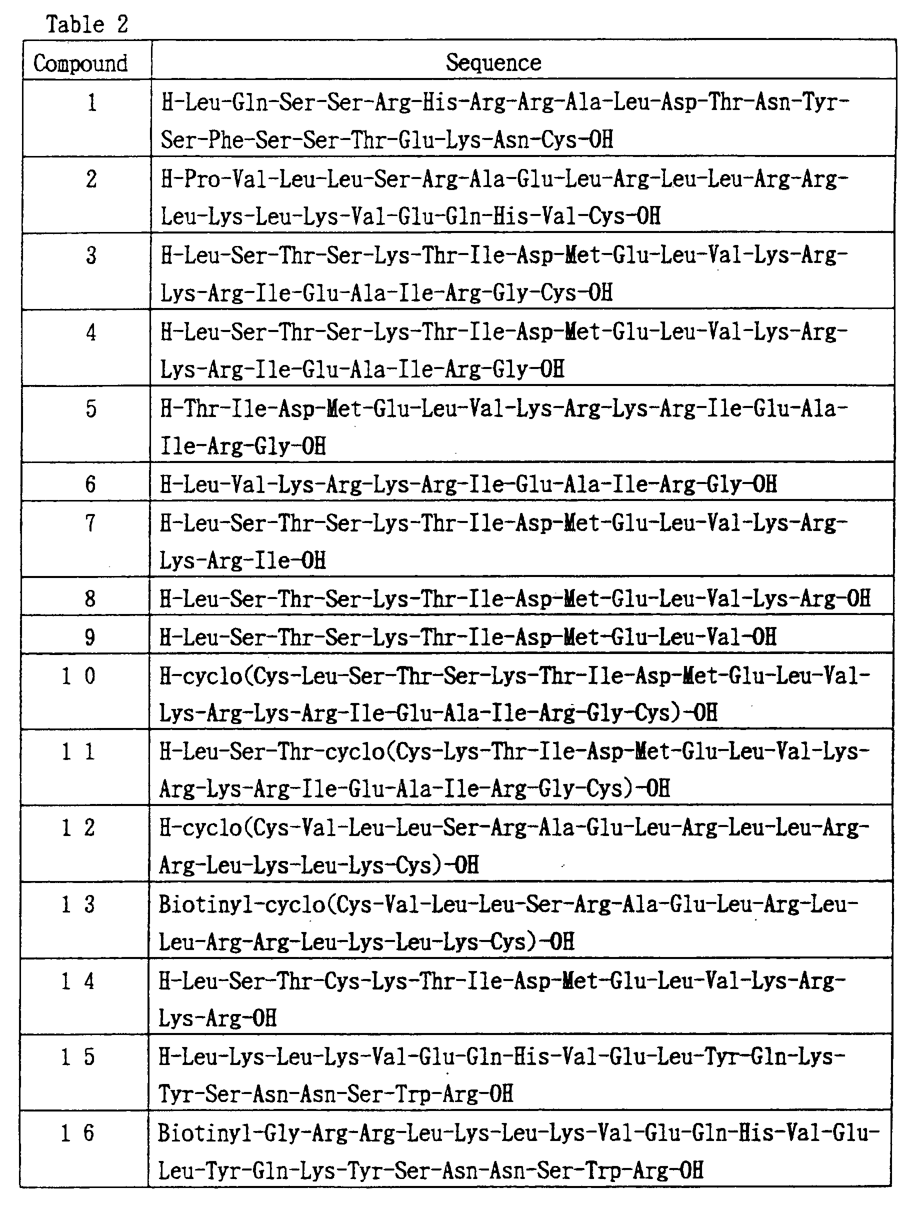

The method of screening a compound to be used for the treatment or prevention of TGF-β-related diseases is described below which comprises measuring the amount of latent TGF-β bound to animal cells after addition of latent TGF-β to said cells, measuring the amount of latent TGF-β bound to animal cells after addition of latent TGF-β and a compound to be evaluated to said cells, and evaluating the inhibiting activity or promoting activity of said compound on the binding of latent TGF-β to animal cells from the change in the amount of latent TGF-β bound to animal cells caused by the addition of said compound.

Either a synthetic compound or a natural substance can be subjected to screening according to this method without any specific restriction. For example, a natural or synthetic peptide, a peptide obtained by hydrolyzing a natural protein with an enzyme, etc. can be evaluated.

The latent TGF-β may be those extracted and purified from animal cells, for example, by the method of Okada, et al. [Journal of Biochemistry, 106, 304 (1989)] or those produced by recombinant DNA techniques [Journal of Biological Chemistry, 271, 29891 (1996)]. Animal cells suitable for use in this method are those to which latent TGF-β can be bound. Examples of such cells are platelets, vascular smooth muscle cells, capillary endothelial cells, aortic endothelial cells, fibroblasts, epithelial cells and macrophages. The cells may be those isolated and purified from animals such as a human, a cow, a pig and a rat, or cultured cells derived from such cells. The cells can be isolated and purified by the method of Okada, et al. [Journal of Biochemistry, 106, 304 (1989)], or the like. The binding of latent TGF-β to cells can be carried out, for example, by first culturing cells in a medium and adding latent TGF-β thereto, followed by incubation, and then washing said cells and measuring the amount of latent TGF-β bound to the cells.

It is preferred to use latent TGF-β which has been ¹²⁵I-labeled according to the chloramine T method [Molecular & Cellular Biology, 2, 599 (1982)], or the like. The amount of latent TGF-β bound to cells can be determined by measuring the radioactivity.

The amount of latent TGF-β bound to cells can also be determined by measuring the amount of active TGF-β because latent TGF-β is activated by being bound to the cells. The determination of active TGF-β can be carried out by any method. For example, the determination can be carried out by methods such as enzyme immunoassay directly using an anti-TGF-β antibody [Methods in Enzymology, 198, 303 (1991)] and the luciferase assay system of Abe, et al. [Analytical Biochemistry, 216, 276 (1994)], or by measuring the degree of migration of vascular endothelial cells [Journal of Cell Biology, 123, 1249 (1993)], growth of vascular smooth muscle cells [Tohoku Journal of Experimental Medicine, 179, 23 (1996)], growth inhibition of various cancer cells [Journal of Clinical Investigation, 87, 277 (1991), Endocrinology, 128, 1981 (1991)] and growth inhibition of mink lung epithelial cells [Methods in Enzymology, 198, 317 (1991)].

The judgment as to whether or not a compound to be evaluated is useful for the treatment or prevention of TGF-β-related diseases is made from the difference between the amount of latent TGF-β bound to the cells or active TGF-β in the absence of said compound and that in the presence of said compound. The desired compound can be preferably obtainable by screening a compound, for example, which increases or decreases the amount of active TGF-β by 10% or more when added at a concentration of 1 mM compared with that measured without addition of the compound.

The method of screening a compound to be used for the treatment or prevention of TGF-β-related diseases is described below which comprises measuring the amount of TGF-β after addition of a peptide shown by Compound (I) or a pharmaceutically acceptable salt thereof to animal cells, measuring the amount of TGF-β after addition of a compound to be evaluated and a peptide shown by Compound (I) or a pharmaceutically acceptable salt thereof to animal cells, and evaluating the inhibiting activity or promoting activity of said compound on the conversion of latent TGF-β into TGF-β from the change in the amount of TGF-β caused by the addition of said compound.

Either a synthetic compound or a natural substance can be subjected to screening according to this method without any specific restriction. For example, a natural or synthetic peptide, a peptide obtained by hydrolyzing a natural protein with an enzyme, etc. can be evaluated.

Cells suitable for use in this mthod are those which secrete latent TGF-β themselves. Such cell lines are preferred because the desired compound can be selected without addition of latent TGF-β to the test system. Examples of the cells which secrete latent TGF-β are vascular endothelial cells, vascular smooth muscle cells and macrophages, and cultured cells derived therefrom.

The amount of active TGF-β can be determined by any method, for example, the methods mentioned above.

The compounds selected according to the present invention include not only the above-defined peptides but also all compounds which have an activity to promote the release of active TGF-β from latent TGF-β or an activity to promote the binding of latent TGF-β to a cell membrane.

The compounds obtainable according to the screening methods of the present invention, Compounds (I) and pharmaceutically acceptable salts thereof can be used for treating or preventing diseases such as cancer, diabetic retinopathy, atherosclerosis, bone fracture, myocardial infarction, myocardial disorder after ischemia reperfusion, cerebral infarction, retinal detachment, glomerulonephritis, diabetic nephropathy, renal graft rejection, HIV nephropathy, sudden pulmonary fibrosis, autoimmune pulmonary fibrosis, hepatic cirrhosis, venous constrictive hepatopathy (often occurring after treatments of cancer), systemic sclerosis, keloid, eosinophilia-muscle ache syndrome, re-stricture after angioplasty, intraocular fibrosis, rheumatic arthritis and nasal polyp. Specifically, they can be preferably used as anti-fibrosis agents, anti-tumor agents and anti-employed as such or in various administration forms. For example, a pharmaceutical composition which is appropriate as an injection can be prepared by dissolving a compound obtained according to the screening method of the present invention, Compound (I) or a pharmaceutically acceptable salt thereof in physiological saline, or an aqueous solution of glucose, lactose or mannitol. A powdery composition for injection can be prepared by freeze-drying a compound obtained according to the screening method of the present invention, Compound (I) or a pharmaceutically acceptable salt thereof and adding sodium chloride thereto. These pharmaceutical compositions may contain as may be appropriate an additive known in the pharmaceutical field, for example, a pharmaceutically acceptable salt.

A pharmaceutical composition for oral administration such as a tablet, granule, powder or syrup can be prepared by mixing a compound obtained according to the screening method of the present invention, Compound (I) or a pharmaceutically acceptable salt thereof with an appropriate excipient, disintegrating agent, binder, lubricant, or the like. Further, a suppository for rectal administration can be prepared by mixing a compound obtained according to the screening method of the present invention, Compound (I) or a pharmaceutically acceptable salt thereof with a conventional carrier.

The effective dose will vary depending upon the mode of administration, the kind of a compound obtained according to the screening method of the present invention, Compound (I) or a pharmaceutically acceptable salt thereof, the age and symptoms of a patient, etc. The mode of administration can also be changed according to the symptoms and the dose. For example, a compound obtained according to the screening method of the present invention, Compound (I) or a pharmaceutically acceptable salt thereof can be administered in a daily dose of 0.00001-100 mg/kg, preferably 0.01-10 mg/kg.

### Brief Description of the Drawings

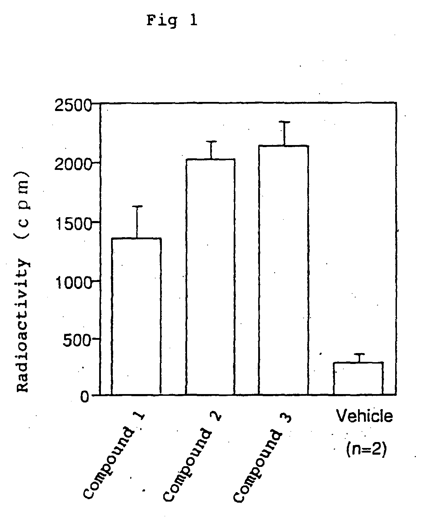

Fig. 1 shows the degree of binding of active TGF-β to porcine vascular smooth muscle cells (PSMC) as determined by measuring the radioactivity. "Vehicle" lane shows the radioactivity when a solution without a test compound was added, and the other lanes show the radioactivity when the respective test compound was added at a concentration of 100 µg/ml.

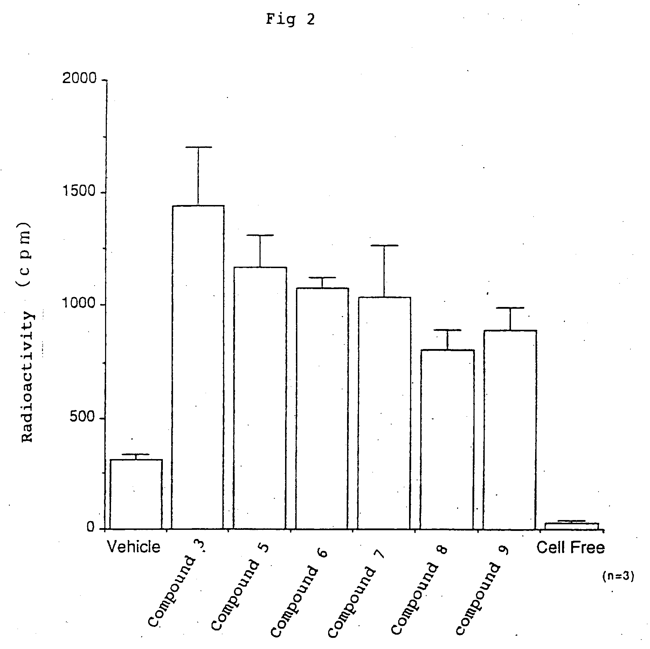

Fig. 2 shows the degree of binding of active TGF-β to bovine vascular smooth muscle cells (BSMC) as determined by measuring the radioactivity. "Vehicle" lane shows the radioactivity when a solution without a test compound was added, "cell free" lane shows the radioactivity when ¹²⁵I-LLTGF-β alone was added to a plate without a cell, and the other lanes show the radioactivity when the respective test compound was added at a concentration of 100 µg/ml.

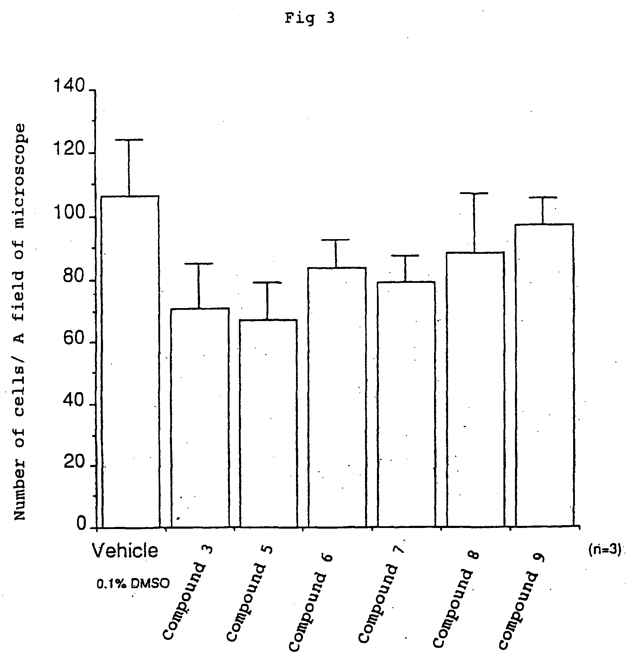

Fig. 3 shows the migration inhibitory activity of active TGF-β on bovine capillary endothelial cells (BCEC) as determined by counting the number of the cells which migrated into a field of a microscope. "Vehicle" lane shows the number of the cells when a solution without a test compound was added, and the other lanes show the number of the cells when the respective test compound was added at a concentration of 50 µg/ml.

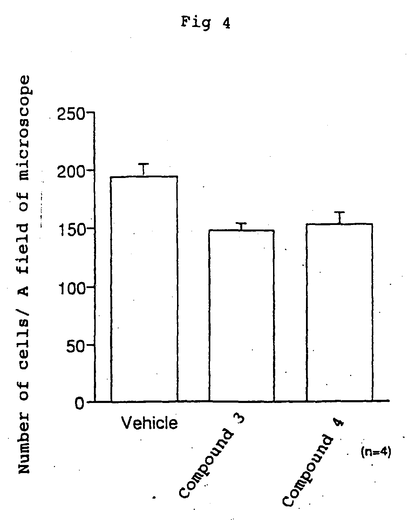

Fig. 4 shows the migration inhibitory activity of active TGF-β on bovine capillary endothelial cells (BCEC) as determined by counting the number of the cells which migrated into a field of a microscope. "Vehicle" lane shows the number of the cells when a solution without a test compound was added, and the other lanes show the number of the cells when the respective test compound was added at a concentration of 100 µg/ml.

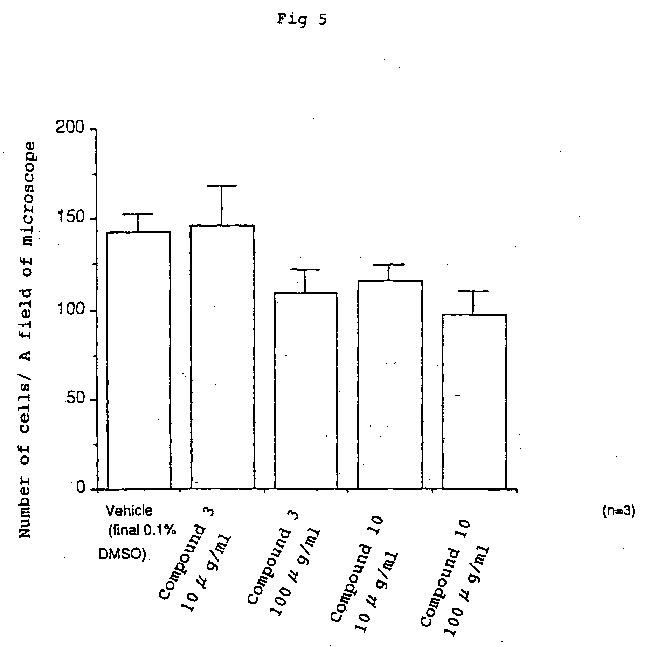

Fig. 5 shows the migration inhibitory activity of active TGF-β on bovine capillary endothelial cells (BCEC) as determined by counting the number of the cells which migrated into a field of a microscope. "Vehicle" lane shows the number of the cells when a solution without a test compound was added, and the other lanes show the number of the cells when the respective test compound was added at the concentration indicated.

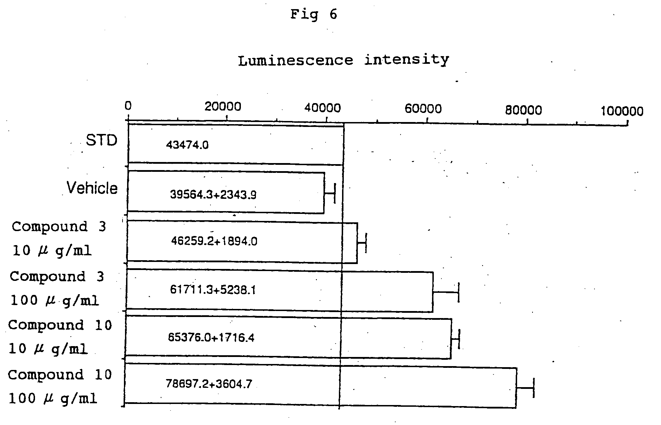

Fig. 6 shows the luminescence intensity as measured by the luciferase assay system for the determination of the amount of active TGF-β. "STD" lane shows the luminescence intensity at stationary state, "vehicle" lane shows the luminescence intensity when a solution without a test compound was added, and the other lanes show the luminescence intensity when the respective compound was added at the concentration indicated.

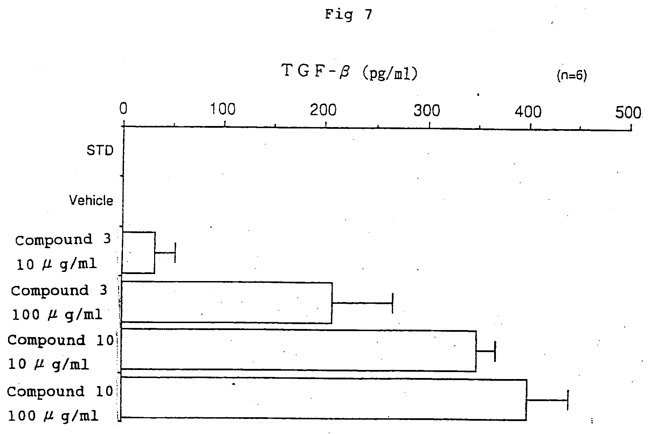

Fig. 7 shows the amount of active TGF-β converted from the luminescence intensity shown in Fig. 6. Each lane has the same significance as that in Fig. 6.

### Best Modes for Carrying Out the Invention

The physicochemical properties of the compounds in Examples below were determined according to the following methods. Mass spectrometric analysis was carried out according to the FAB method using JEOL JMS-SX102A. Amino acid analysis was carried out according to the method of Bidlingmeyer, B.A., et al. [Journal of Chromatography, 336, 93 (1984)]. Hydrolysis was carried out in hydrochloric acid vapor at 110°C for 22 hours. The amino acid compositions of the resulting hydrolyzates were analyzed with Pico Tag amino acid analyzer (Waters Associates).

### Example 1 Synthesis of Compound 1 (SEQ ID NO: 1) (H-Leu-Gln-Ser-Ser-Arg-His-Arg-Arg-Ala-Leu-Asp-Thr-Asn-Tyr-Ser-Phe-Ser-Ser-Thr-Glu-Lys-Asn-Cys-OH)

A carrier resin (Wang resin, 123 mg) combined with 62.5 µmmol of Fmoc-Cys(Trt) was put in a reactor of an automatic synthesizer (ABI, model 430A) and the following treatments were carried out by the Fmoc method according to the synthesis program developed by ABI.
(a) To the carrier resin was added a 20% piperidine-NMP solution, and the mixture was stirred for 20 minutes, followed by discharge of said solution.
(b) The carrier resin was washed with NMP for 5 minutes, and the rinsings were discharged. The carrier resin combined with Cys(Trt) without Fmoc group was thus obtained.
(c) A solution previously prepared by stirring 250 µmol (4 equivalents based on the amino acid on the resin) of Fmoc-Asn(Trt)-OH, DCC and HOBt in NMP for 50 minutes was added to the resin, and the resulting mixture was stirred for 60 minutes, followed by discharge of said solution.
(d) The carrier resin was washed with NMP for 3 minutes. Fmoc-Asn(Trt)-Cys(Trt) was thus synthesized on the carrier resin.

Subsequently, deprotection and washing steps (a) and (b) were carried out, and condensation reaction was carried out using Fmoc-Lys(Boc)-OH in step (c), followed by washing step (d) to synthesize Fmoc-Lys(Boc)-Asn(Trt)-Cys(Trt) on the carrier resin. Then, steps (a)-(d) were repeated to obtain the carrier resin to which a protected peptide was bound. In step (c) in the repeated procedures, Fmoc-Glu(Ot-Bu)-OH, Fmoc-Thr(t-Bu)-OH, Fmoc-Ser(t-Bu)-OH, Fmoc-Ser(t-Bu)-OH, Fmoc-Phe-OH, Fmoc-Ser(t-Bu)-OH, Fmoc-Tyr(t-Bu)-OH, Fmoc-Asn(Trt)-OH, Fmoc-Thr(t-Bu)-OH, Fmoc-Asp(Ot-Bu)-OH, Fmoc-Leu-OH, Fmoc-Ala-OH, Fmoc-Arg(Pmc)-OH, Fmoc-Arg(Pmc)-OH, Fmoc-His(Trt)-OH, Fmoc-Arg(Pmc)-OH, Fmoc-Ser(t-Bu)-OH, Fmoc-Ser(t-Bu)-OH, Fmoc-Gln(Trt)-OH and Fmoc-Leu-OH were used in turn. After deprotection and washing steps (a) and (b) were carried out, the carrier resin was washed successively with methanol and butyl ether, followed by drying under reduced pressure for 12 hours to obtain 310.3 mg of the carrier resin to which a side-chain-protected peptide was bound. To the obtained carrier resin was added 10 ml of a mixture of TFA (82.5%), thioanisole (5%), water (5%), ethyl methyl sulfide (3%), 1,2-ethanedithiol (2.5%) and thiophenol (2%), and the resulting mixture was allowed to stand at room temperature for 8 hours to remove the side-chain-protecting groups and to cleave the peptide from the resin. After the resin was separated by filtration, the filtrate was concentrated to about 2 ml under reduced pressure, and about 10 ml of ether was added thereto. The deposited precipitate was collected by centrifugation and decantation to obtain 145 mg of the crude peptide. A part of the obtained crude peptide (70 mg) was dissolved in 2 M acetic acid and then purified by HPLC using a reversed-phase column (Shiseido Co., Ltd., CAPCELL PAK C18 30 mm I.D. x 25 mm). Elution was carried out with a linear concentration gradient by adding 90% aqueous acetonitrile containing 0.1% TFA to 0.1% aqueous TFA, followed by detection at 220 nm to give a fraction containing Compound 1. The obtained fraction was lyophilized to give 25.5 mg of Compound 1.
Mass spectrum [FABMS]: m/z=2701.1 (M+H⁺)
Amino acid analysis: Asx 3.1 (3), Glx 2.1 (2), Ser 5.0 (5), His 1.0 (1), Arg 2.9 (3), Thr 2.0 (2), Ala 1.0 (1), Tyr 1.0 (1), Leu 1.9 (2), Phe 1.0 (1), Lys 1.1 (1), Cys 1.1 (1)

### Example 2 Synthesis of Compound 2 (SEQ ID NO: 2) (H-Pro-Val-Leu-Leu-Ser-Arg-Ala-Glu-Leu-Arg-Leu-Leu-Arg-Arg-Leu-Lys-Leu-Lys-Val-Glu-Gln-His-Val-Cys-OH)

Condensation was carried out in the same manner as in Example 1 using 181.8 mg of a carrier resin (Wang resin) combined with 100 µmol of Fmoc-Cys(Trt) as a starting material, and using Fmoc-Val-OH, Fmoc-His(Trt)-OH, Fmoc-Gln(Trt)-OH, Fmoc-Glu(Ot-Bu)-OH, Fmoc-Val-OH, Fmoc-Lys(Boc)-OH, Fmoc-Leu-OH, Fmoc-Lys(Boc)-OH, Fmoc-Leu-OH, Fmoc-Arg(Pmc)-OH, Fmoc-Arg(Pmc)-OH, Fmoc-Leu-OH, Fmoc-Leu-OH, Fmoc-Arg(Pmc)-OH, Fmoc-Leu-OH, Fmoc-Glu(Ot-Bu)-OH, Fmoc-Ala-OH, Fmoc-Arg(Pmc)-OH, Fmoc-Ser(t-Bu)-OH, Fmoc-Leu-OH, Fmoc-Leu-OH, Fmoc-Val-OH and Fmoc-Pro-OH in turn. The condensation product was washed and dried to obtain 568.4 mg of the carrier resin to which a side-chain-protected peptide was bound. Removal of the side-chain-protecting groups and cleavage of the peptide from the resin were carried out in the same manner as in Example 1 to obtain 320 mg of the crude peptide. The obtained crude peptide was purified by HPLC using a reversed-phase column (Shiseido Co., Ltd., CAPCELL PAK C18 30 mm I. D. x 250 mm) to give 26.4 mg of Compound 2.
Mass spectrum [FABMS]: m/z=2870.4 (M+H⁺)
Amino acid analysis: Glx 3.0 (3), Ser 1.1 (1), His 1.0 (1), Arg 3.8 (4), Ala 1.1 (1), Pro 0.9 (1), Val 2.6 (3), Leu 7.3 (7), Lys 2.1 (2), Cys 1.2 (1)

### Example 3 Synthesis of Compound 3 (SEQ ID NO: 3) (H-Leu-Ser-Thr-Ser-Lys-Thr-Ile-Asp-Met-Glu-Leu-Val-Lys-Arg-Lys-Arg-Ile-Glu-Ala-Ile-Arg-Gly-Cys-OH)

A carrier resin (Wang resin, 60 mg) combined with 33 µmol of Fmoc-Cys(Trt) was put in a reactor of an automatic synthesizer (Shimadzu Corporation, PSSM-8), and the following treatments were carried out according to the synthesis program.
(a) The carrier resin was washed with 500 µl of DMF for 3 minutes, and the rinsings were discharged.
(b) To the carrier resin was added 500 µl of a 30% piperidine-DMF solution, and the mixture was stirred for 4 minutes, followed by discharge of said solution. The same treatment was repeated.
(c) The carrier resin was washed with 500 µl of DMF for one minute, and the rinsings were discharged. The same treatment was repeated 5 times. The carrier resin combined with Cys(Trt) without Fmoc was thus obtained.
(d) DMF (1155 µl) containing 330 µmol of Fmoc-Gly-OH, 330 µmol of PyBOP, 330 µmol of HOBt monohydrate and 495 µmol of NMM was stirred for 3 minutes. The resulting solution was added to the carrier resin and the mixture was stirred for 30 minutes, followed by discharge of the solution.
(e) The carrier resin was washed with 500 µl of DMF for one minute. The same treatment was repeated 5 times. Fmoc-Gly-Cys(Trt) was thus synthesized on the carrier resin.

Subsequently, washing and deprotection steps (a)-(c) were carried out, and condensation reaction was carried out using Fmoc-Arg(Pmc)-OH in step (d), followed by washing step (e) to synthesize Fmoc-Arg(Pmc)-Gly-Cys(Trt) on the carrier resin. Then, steps (a)-(e) were repeated to obtain the carrier resin to which a protected peptide was bound. In step (d) in the repeated procedures, Fmoc-Ile-OH, Fmoc-Ala-OH, Fmoc-Glu(Ot-Bu)-OH, Fmoc-Ile-OH, Fmoc-Arg(Pmc)-OH, Fmoc-Lys(Boc)-OH, Fmoc-Arg(Pmc)-OH, Fmoc-Lys(Boc)-OH, Fmoc-Val-OH, Fmoc-Leu-OH, Fmoc-Glu(Ot-Bu)-OH, Fmoc-Met-OH, Fmoc-Asp(Ot-Bu)-OH, Fmoc-Ile-OH, Fmoc-Thr(t-Bu)-OH, Fmoc-Lys(Boc)-OH, Fmoc-Ser(t-Bu)-OH, Fmoc-Thr(t-Bu)-OH, Fmoc-Ser(t-Bu)-OH and Fmoc-Leu-OH were used in turn. Subsequently, washing and deprotection steps (a)-(c) were carried out, and the carrier resin was washed with 500 µl of DMF for one minute. The same treatment was repeated 5 times, and the carrier resin was washed successively with methanol and butyl ether, followed by drying under reduced pressure for 12 hours to obtain the carrier resin to which a side-chain-protected peptide was bound. Removal of the side-chain-protecting groups and cleavage of the peptide from the resin were carried out in the same manner as in Example 1 to obtain 103.2 mg of the crude peptide. The obtained crude peptide was purified by HPLC using a reversed-phase column (YMC, YMC-Pack ODS-AM 30 mm I.D. x 250 mm) to give 25.4 mg of Compound 3.
Mass spectrum [FABMS]: m/z=2648.1 (M+H⁺)
Amino acid analysis: Asx 1.1 (1), Glx 2.1 (2), Ser 1.9 (2), Gly 1.1 (1), Arg 2.5 (3), Thr 1.8 (2), Ala 1.1 (1), Val 0.9 (1), Met 1.0 (1), Ile 3.0 (3), Leu 2.1 (2), Lys 3.1 (3), Cys 1.2 (1)

### Example 4 Synthesis of Compound 4 (SEQ ID NO: 4) (H-Leu-Ser-Thr-Ser-Lys-Thr-Ile-Asp-Met-Glu-Leu-Val-Lys-Arg-Lys-Arg-Ile-Glu-Ala-Ile-Arg-Gly-OH)

Condensation was carried out in the same manner as in Example 1 using 60 mg of a carrier resin (Wang resin) combined with 33 µmol of Fmoc-Gly as a starting material, and using Fmoc-Arg(Pmc)-OH, Fmoc-Ile-OH, Fmoc-Ala-OH, Fmoc-Glu(Ot-Bu)-OH, Fmoc-Ile-OH, Fmoc-Arg(Pmc)-OH, Fmoc-Lys(Boc)-OH, Fmoc-Arg(Pmc)-OH, Fmoc-Lys(Boc)-OH, Fmoc-Val-OH, Fmoc-Leu-OH, Fmoc-Glu(Ot-Bu)-OH, Fmoc-Met-OH, Fmoc-Asp(Ot-Bu)-OH, Fmoc-Ile-OH, Fmoc-Thr(t-Bu)-OH, Fmoc-Lys(Boc)-OH, Fmoc-Ser(t-Bu)-OH, Fmoc-Thr(t-Bu)-OH, Fmoc-Ser(t-Bu)-OH and Fmoc-Leu-OH in turn. The condensation product was washed and dried to obtain the carrier resin to which a side-chain-protected peptide was bound. Removal of the side-chain-protecting groups and cleavage of the peptide from the resin were carried out in the same manner as in Example 1 to obtain 115.3 mg of the crude peptide. The obtained crude peptide was purified by HPLC using a reversed-phase column (YMC, YMC-Pack ODS-AM 30 mm I.D. x 250 mm) to give 63.3 mg of Compound 4.
Mass spectrum [FABMS]: m/z=2545.1 (M+H⁺)
Amino acid analysis: Asx 1.1 (1), Glx 2.1 (2), Ser 1.9 (2), Gly 1.1 (1), Arg 2.6 (3), Thr 1.9 (2), Ala 1.1 (1), Val 0.9 (1), Met 0.9 (1), Ile 3.0 (3), Leu 2.1 (2), Lys 3.0 (3)

### Example 5 Synthesis of Compound 5 (SEQ ID NO: 5) (H-Thr-Ile-Asp-Met-Glu-Leu-Val-Lys-Arg-Lys-Arg-Ile-Glu-Ala-Ile-Arg-Gly-OH)

The following treatments were carried out using 51.0 mg of a carrier resin (Wang resin) combined with 25 µmol of Fmoc-Gly as a starting material by the use of a peptide synthesizer (ACT, ACT357).
(a) The carrier resin was washed with 1 ml of DMF for 30 seconds, and the rinsings were discharged.
(b) To the carrier resin was added 1 ml of a 25% piperidine-DMF solution and the mixture was stirred for 2 minutes, followed by discharge of said solution. To the carrier resin was added again 1 ml of a 25% piperidine-DMF solution and the mixture was stirred for 10 minutes, followed by discharge of said solution.
(c) The carrier resin was washed with DMF for 12 seconds, followed by discharge of the rinsings. The same treatment was repeated 7 times. The carrier resin combined with Gly without Fmoc was thus obtained.
(d) To the carrier resin were added 125 µl of DMF, 500 µl of NMP solution containing 0.25 M Fmoc-Arg(Pmc)-OH and 0.25 M HOBt monohydrate, 500 µl of DMF solution containing 0.25 M PyBOP and 125 µl of DMF solution containing 2.0 M NMM. After stirring for 60 minutes, the solutions were discharged.
(e) The reactor was washed with 500 µl of DMF, and then with 1 ml of DMF for 30 seconds with stirring, followed by further washing with 500 µl of DMF. Fmoc-Arg(Pmc)-Gly was thus synthesized on the carrier resin.

Subsequently, washing and deprotection steps (a)-(c) were carried out, and condensation reaction was carried out using a solution containing Fmoc-Ile-OH in place of Fmoc-Arg(Pmc)-OH in step (d), followed by washing step (e) to synthesize Fmoc-Ile-Arg(Pmc)-Gly on the carrier resin. Then, condensation was carried out using, in step (d), Fmoc-Ala-OH, Fmoc-Glu(Ot-Bu)-OH, Fmoc-Ile-OH, Fmoc-Arg(Pmc)-OH, Fmoc-Lys(Boc)-OH, Fmoc-Arg(Pmc)-OH, Fmoc-Lys(Boc)-OH, Fmoc-Val-OH, Fmoc-Leu-OH, Fmoc-Glu(Ot-Bu)-OH, Fmoc-Met-OH, Fmoc-Asp(Ot-Bu)-OH, Fmoc-Ile-OH and Fmoc-Thr(t-Bu)-OH in turn. The condensation product was washed and dried to obtain 132.5 mg of the carrier resin (Wang resin) to which a side-chain-protected peptide was bound. Removal of the side-chain-protecting groups and cleavage of the peptide from the resin were carried out in the same manner as in Example 1 to obtain 58.3 mg of the crude peptide. The obtained crude peptide was purified by HPLC using a reversed-phase column (YMC, YMC-Pack ODS-AM 30 mm I.D. x 250 mm) in the same manner as in Example 1 to give 22.6 mg of Compound 5.
Mass spectrum [FABMS]: m/z=2029.2 (M+H⁺)
Amino acid analysis: Asx 1.0 (1), Glx 2.0 (2), Gly 1.0 (1), Arg 3.0 (3), Thr 1.0 (1), Ala 1.1 (1), Val 0.9 (1), Met 1.1 (1), Ile 2.9 (3), Leu 1.1 (1), Lys 2.0 (2)

### Example 6 Synthesis of Compound 6 (SEQ ID NO: 6) (H-Leu-Val-Lys-Arg-Lys-Arg-Ile-Glu-Ala-Ile-Arg-Gly-OH)

Condensation was carried out in the same manner as in Example 5 using 51.0 mg of a carrier resin (Wang resin) combined with 25.0 µmol of Fmoc-Gly as a starting material, and using Fmoc-Arg(Pmc)-OH, Fmoc-Ile-OH, Fmoc-Ala-OH, Fmoc-Glu(Ot-Bu)-OH, Fmoc-Ile-OH, Fmoc-Arg(Pmc)-OH, Fmoc-Lys(Boc)-OH, Fmoc-Arg(Pmc)-OH, Fmoc-Lys(Boc)-OH, Fmoc-Val-OH and Fmoc-Leu-OH in turn. The condensation product was washed and dried to obtain 112.2 mg of the carrier resin to which a side-chain-protected peptide was bound. Removal of the side-chain-protecting groups and cleavage of the peptide from the resin were carried out in the same manner as in Example 1 to obtain 48.7 mg of the crude peptide. The obtained crude peptide was dissolved in 1 ml of TFA, and the resulting solution was added dropwise into 50 ml of ether. The deposited precipitate was separated by filtration and then dried to give 35.5 mg of Compound 6.
Mass spectrum [FABMS]: m/z=1439.0 (M+H⁺)
Amino acid analysis: Glx 1.1 (1), Gly 1.0 (1), Arg 3.0 (3), Ala 1.1 (1), Val 0.8 (1), Ile 1.9 (2), Leu 1.0 (1), Lys 2.0 (2)

### Example 7 Synthesis of Compound 7 (SEQ ID NO: 7) (H-Leu-Ser-Thr-Ser-Lys-Thr-Ile-Asp-Met-Glu-Leu-Val-Lys-Arg-Lys-Arg-Ile-OH)

Condensation was carried out in the same manner as in Example 5 using 56.8 mg of a carrier resin (Wang resin) combined with 25.0 µmol of Fmoc-Ile as a starting material, and using Fmoc-Arg(Pmc)-OH, Fmoc-Lys(Boc)-OH, Fmoc-Arg(Pmc)-OH, Fmoc-Lys(Boc)-OH, Fmoc-Val-OH, Fmoc-Leu-OH, Fmoc-Glu(Ot-Bu)-OH, Fmoc-Met-OH, Fmoc-Asp(Ot-Bu)-OH, Fmoc-Ile-OH, Fmoc-Thr(t-Bu)-OH, Fmoc-Lys(Boc)-OH, Fmoc-Ser(t-Bu)-OH, Fmoc-Thr(t-Bu)-OH, Fmoc-Ser(t-Bu)-OH and Fmoc-Leu-OH in turn. The condensation product was washed and dried to obtain 280.9 mg of the carrier resin to which a side-chain-protected peptide was bound. Removal of the side-chain-protecting groups and cleavage of the peptide from the resin were carried out in the same manner as in Example 1 to obtain 123.1 mg of the crude peptide. The obtained crude peptide was purified by HPLC using a reversed-phase column (YMC, YMC-Pack ODS-AM 30 mm I.D. x 250 mm) in the same manner as in Example 1 to give 26.7 mg of Compound 7.
Mass spectrum [FABMS]: m/z=2019.2 (M+H⁺)
Amino acid analysis: Asx 1.1 (1), Glx 1.1 (1), Ser 1.7 (2), Arg 2.2 (2), Thr 1.7 (2), Val 1.0 (1), Met 1.0 (1), Ile 2.1 (2), Leu 2.1 (2), Lys 3.1 (3)

### Example 8 Synthesis of Compound 8 (SEQ ID NO: 8) (H-Leu-Ser-Thr-Ser-Lys-Thr-Ile-Asp-Met-Glu-Leu-Val-Lys-Arg-OH)

Condensation was carried out in the same manner as in Example 5 using 48.1 mg of a carrier resin (Wang resin) combined with 25.0 µmol of Fmoc-Arg(Pmc) as a starting material, and using Fmoc-Lys(Boc)-OH, Fmoc-Val-OH, Fmoc-Leu-OH, Fmoc-Glu(Ot-Bu)-OH, Fmoc-Met-OH, Fmoc-Asp(Ot-Bu)-OH, Fmoc-Ile-OH, Fmoc-Thr(t-Bu)-OH, Fmoc-Lys(Boc)-OH, Fmoc-Ser(t-Bu)-OH, Fmoc-Thr(t-Bu)-OH, Fmoc-Ser(t-Bu)-OH and Fmoc-Leu-OH in turn. The condensation product was washed and dried to obtain 79.3 mg of the carrier resin to which a side-chain-protected peptide was bound. Removal of the side-chain-protecting groups and cleavage of the peptide from the resin were carried out in the same manner as in Example 1 to obtain 18.3 mg of the crude peptide. The obtained crude peptide was purified by HPLC using a reversed-phase column (YMC, YMC-Pack ODS-AM 30 mm I.D. x 250 mm) in the same manner as in Example 1 to give 4.7 mg of Compound 8.
Mass spectrum [FABMS]: m/z=1621.0 (M+H⁺)
Amino acid analysis: Asx 1.0 (1), Glx 1.1 (1), Ser 1.8 (2), Arg 1.1 (1), Thr 1.9 (2), Val 0.9 (1), Met 1.0 (1), Ile 1.0 (1), Leu 2.1 (2), Lys 2.0 (2)

### Example 9 Synthesis of Compound 9 (SEQ ID NO: 9) (H-Leu-Ser-Thr-Ser-Lys-Thr-Ile-Asp-Met-Glu-Leu-Val-OH)

Condensation was carried out in the same manner as in Example 5 using 48.1 mg of a carrier resin (Wang resin) combined with 25.0 µmol of Fmoc-Val as a starting material and using Fmoc-Leu-OH, Fmoc-Glu(Ot-Bu)-OH, Fmoc-Met-OH, Fmoc-Asp(Ot-Bu)-OH, Fmoc-Ile-OH, Fmoc-Thr(t-Bu)-OH, Fmoc-Lys(Boc)-OH, Fmoc-Ser(t-Bu)-OH, Fmoc-Thr(t-Bu)-OH, Fmoc-Ser(t-Bu)-OH and Fmoc-Leu-OH in turn. The condensation product was washed and dried to obtain 120.3 mg of the carrier resin to which a side-chain-protected peptide was bound. Removal of the side-chain-protecting groups and cleavage of the peptide from the resin were carried out in the same manner as in Example 1, except that a mixture of 90% TFA, 5% 1,2-ethanedithiol and 5% thioanisole was used and the standing time was 2 hours, to obtain 49.0 mg of the crude peptide. The obtained crude peptide was purified by HPLC using a reversed-phase column (YMC, YMC-Pack ODS-AM 30 mm I.D. x 250 mm) in the same manner as in Example 1 to give 20.2 mg of Compound 9.
Mass spectrum [FABMS]: m/z=1336.7 (M+H⁺)
Amino acid analysis: Asx 1.1 (1), Glx 1.1 (1), Ser 1.8 (2), Thr 1.8 (2), Val 1.0 (1), Met 1.0 (1), Ile 1.0 (1), Leu 2.1 (2), Lys 1.0 (1)

### Example 10 Synthesis of Compound 10 (SEQ ID NO: 10] [H-cyclo(Cys-Leu-Ser-Thr-Ser-Lys-Thr-Ile-Asp-Met-Glu-Leu-Val-Lys-Arg-Lys-Arg-Ile-Glu-Ala-Ile-Arg-Gly-Cys)-OH]

Condensation was carried out in the same manner as in Example 3 using 30 mg of a carrier resin (Wang resin) combined with 16.5 µmol of Fmoc-Cys(Trt) as a starting material, and using Fmoc-Gly, Fmoc-Arg(Pmc)-OH, Fmoc-Ile-OH, Fmoc-Ala-OH, Fmoc-Glu(Ot-Bu)-OH, Fmoc-Ile-OH, Fmoc-Arg(Pmc)-OH, Fmoc-Lys(Boc)-OH, Fmoc-Arg(Pmc)-OH, Fmoc-Lys(Boc)-OH, Fmoc-Val-OH, Fmoc-Leu-OH, Fmoc-Glu(Ot-Bu)-OH, Fmoc-Met-OH, Fmoc-Asp(Ot-Bu)-OH, Fmoc-Ile-OH, Fmoc-Thr(t-Bu)-OH, Fmoc-Lys(Boc)-OH, Fmoc-Ser(t-Bu)-OH, Fmoc-Thr(t-Bu)-OH, Fmoc-Ser(t-Bu)-OH, Fmoc-Leu-OH and Fmoc-Cys(Trt)-OH in turn. The condensation product was washed and dried to obtain the carrier resin to which a side-chain-protected peptide was bound. The reaction time for condensation with each amino acid was 60 minutes. Removal of the side-chain-protecting groups and cleavage or the peptide from the resin were carried out in the same manner as in Example 1 to obtain 54.5 mg of the crude peptide. The obtained crude peptide was purified by HPLC using a reversed-phase column (Shiseido Co., Ltd., CAPCELL PAK C18 30 mm I.D. x 250 mm) in the same manner as in Example 1 to give 14.0 mg of an uncyclized form of Compound 10.
Mass spectrum [FABMS]: m/z=2751.7 (M+H⁺)

The obtained compound was dissolved in 10 ml of DMSO, and aqueous ammonia was added thereto to adjust the pH of the solution to 7.1, followed by stirring at room temperature for 29 hours. The resulting solution was lyophilized and then dissolved in a 2 M aqueous solution of acetic acid, followed by freeze-drying to give 13.5 mg of Compound 10.
Mass spectrum [FABMS]: m/z=2749.5 (M+H⁺)
Amino acid analysis: Asx 1.2 (1), Glx 2.2 (2), Ser 2.0 (2), Gly 1.1 (1), Arg 3.0 (3), Thr 2.0 (2), Ala 1.1 (1), Val 1.0 (1), Met 1.1 (1), Ile 3.1 (3), Leu 2.2 (2), Lys 3.1 (3), Cys 1.8 (2)

### Example 11 Synthesis of Compound 11 (SEQ ID NO: 11) [H-Leu-Ser-Thr-cyclo(Cys-Lys-Thr-Ile-Asp-Met-Glu-Leu-Val-Lys-Arg-Lys-Arg-Ile-Glu-Ala-Ile-Arg-Gly-Cys)-OH]

Condensation was carried out in the same manner as in Example 3 using 30 mg of a carrier resin (Wang resin) combined with 16.5 µmol of Fmoc-Cys(Trt) as a starting material, and using Fmoc-Gly, Fmoc-Arg(Pmc)-OH, Fmoc-Ile-OH, Fmoc-Ala-OH, Fmoc-Glu(Ot-Bu)-OH, Fmoc-Ile-OH, Fmoc-Arg(Pmc)-OH, Fmoc-Lys(Boc)-OH, Fmoc-Arg(Pmc)-OH, Fmoc-Lys(Boc)-OH, Fmoc-Val-OH, Fmoc-Leu-OH, Fmoc-Glu(Ot-Bu)-OH, Fmoc-Met-OH, Fmoc-Asp(Ot-Bu)-OH, Fmoc-Ile-OH, Fmoc-Thr(t-Bu)-OH, Fmoc-Lys(Boc)-OH, Fmoc-Cys(Trt)-OH, Fmoc-Thr(t-Bu)-OH, Fmoc-Ser(t-Bu)-OH and Fmoc-Leu-OH in turn. The condensation product was washed and dried to obtain the carrier resin to which a side-chain-protected peptide was bound. The reaction time for condensation with each amino acid was 60 minutes. Removal of the side-chain-protecting groups and cleavage of the peptide from the resin were carried out in the same manner as in Example 1 to obtain 55.2 mg of the crude peptide. The obtained crude peptide was purified by HPLC using a reversed-phase column (Shiseido Co., Ltd., CAPCELL PAK C18 30 mm I.D. x 250 mm) in the same manner as in Example 1 to give 9.7 mg of an uncyclized form of Compound 11.
Mass spectrum [FABMS]: m/z=2664.5 (M+H⁺)

The obtained compound was dissolved in 10 ml of DMSO, and aqueous ammonia was added thereto to adjust the pH of the solution to 7.3, followed by stirring at room temperature for 20 hours. The resulting solution was lyophilized and then dissolved in a 2 M aqueous solution of acetic acid, followed by freeze-drying to give 9.9 mg of Compound 11.
Mass spectrum [FABMS]: m/z=2662.5 (M+H⁺)
Amino acid analysis: Asx 0.9 (1), Glx 2.0 (2), Ser 0.9 (1), Gly 1.1 (1), Arg 3.0 (3), Thr 1.9 (2), Ala 1.1 (1), Val 0.9 (1), Met 1.0 (1), Ile 3.2 (3), Leu 2.1 (2), Lys 2.9 (3), Cys 1.5 (2)

### Example 12 Synthesis of Compound 12 (SEQ ID NO: 12) [H-cyclo(Cys-Val-Leu-Leu-Ser-Arg-Ala-Glu-Leu-Arg-Leu-Leu-Arg-Arg-Leu-Lys-Leu-Lys-Cys)-OH]

The following treatments were carried out using 250 mg of a carrier resin (Cl-Trt resin) combined with 140 µmol of H-Cys(Trt) as a starting material by the use of a peptide synthesizer (ACT, ACT357).
(a) The carrier resin was washed with 2.5 ml of DMF for 3 minutes, and the rinsings were discharged. The same treatment was repeated.
(b) To the carrier resin were added 250 µl of DMF, 1.4 ml of NMP solution containing 0.5 M Fmoc-Lys(Boc)OH and 0.5 M HOBt monohydrate and 1.4 ml of NMP solution containing 0.5 M DIPC. After stirring for 40 minutes, the solutions were discharged.
(c) The carrier resin was washed with 2.5 ml of DMF for one minute, and the rinsings were discharged. The same treatment was repeated twice.
(d) To the carrier resin were added 250 µl of DMF, 1.4 ml of NMP solution containing 0.5 M Fmoc-Lys(Boc)-OH and 0.5 M HOBt monohydrate, 1.4 ml of DMF solution containing 0.5 M HBTU, and 0.7 ml of NMP solution containing 2.0 M DIEA. After stirring for 20 minutes, the solutions were discharged.
(e) The same treatment as in (c) was carried out. Fmoc-Lys(Boc)-Cys(Trt) was thus synthesized on the carrier resin.
(f) To the carrier resin was added 2.5 ml of DMF containing 25% piperidine, and the resulting mixture was stirred for two minutes, followed by discharge of said solution. To the carrier resin was added again 2.5 ml of the same solution, and the resulting mixture was stirred for 10 minutes, followed by discharge of said solution.
(g) The carrier resin was washed with 2.5 ml of DMF for one minute, and the rinsings were discharged. The same treatment was repeated 7 times. Thus, the carrier resin combined with Lys(Boc)-Cys(Trt) without Fmoc was obtained.

Subsequently, condensation reaction was carried out using a solution containing Fmoc-Leu-OH in place of Fmoc-Lys(Boc)-OH in steps (a)-(e), followed by deprotection and washing steps (f) and (g) to synthesize Leu-Lys(Boc)-Cys(Trt) on the carrier resin. Then, steps (a)-(e) were repeated using Fmoc-Lys(Boc)-OH, Fmoc-Leu-OH, Fmoc-Arg(Pmc)-OH, Fmoc-Arg(Pmc)-OH, Fmoc-Leu-OH, Fmoc-Leu-OH, Fmoc-Arg(Pmc)-OH, Fmoc-Leu-OH, Fmoc-Glu(OtBu)-OH, Fmoc-Ala-OH, Fmoc-Arg(Pmc)-OH, Fmoc-Ser(tBu)-OH, Fmoc-Leu-OH, Fmoc-Leu-OH, Fmoc-Val-OH and Fmoc-Cys(Trt)-OH in turn. The condensation product was washed and dried to obtain the carrier resin to which a side-chain-protected peptide was bound. Removal of the side-chain-protecting groups and cleavage of the peptide from the resin were carried out in the same manner as in Example 1 using 4/5 the amount of the obtained resin to give 137.2 mg of the crude peptide. The obtained crude peptide and 100 mg of DTT were dissolved in 2 ml of DMF, and the solution was allowed to stand at 50°C for one hour. The resulting solution was purified by HPLC using a reversed-phase column (Shiseido Co., Ltd., CAPCELL PAK C18 30 mm I.D. X 250 mm) in the same manner as in Example 1 to give 12.2 mg of a peptide having two free SH groups.
Mass spectrum [FABMS]: m/z=2284.3 (M+H⁺)

The obtained peptide was dissolved in 5 ml of a 2 M aqueous solution of acetic acid. After the resulting solution was diluted with water to 50 ml, dilute aqueous ammonia was added thereto to adjust the pH of the solution to 5.7. To the resulting mixture was added 0.5 ml of a 0.1 M aqueous solution of K₃Fe(CN)₆, followed by stirring at room temperature for 2.5 hours. After addition of 1 ml of acetic acid, the reaction mixture was purified by HPLC using a reversed-phase column (Shiseido Co., Ltd., CAPCELL PAK C18 30 mm I.D. x 250 mm) in the same manner as in Example 1 to give 7.3 mg of Compound 12.
Mass spectrum [FABMS]: m/z=2282.5 (M+H⁺)
Amino acid analysis: Glx 1.0 (1), Ser 1.0 (1), Arg 3.9 (4), Ala 1.1 (1), Val 0.7 (1), Leu 7.2 (7), Lys 2.1 (2), Cys 2.7 (2)

### Example 13 Synthesis of Compound 13 (SEQ ID NO: 13) [Biotinyl-cyclo(Cys-Val-Leu-Leu-Ser-Arg-Ala-Glu-Leu-Arg-Leu-Leu-Arg-Arg-Leu-Lys-Leu-Lys-Cys)-OH]

After 1/5 the amount of the carrier resin with the side-chain-protected peptide obtained in Example 12 was washed with 1 ml of DMF, 440 µl of DMF suspension containing 68.4 mg of D-biotin (Nakalai Tesque, Inc.) and NMP solution containing 0.5 M DIPC were added thereto, followed by stirring at room temperature for 2 days. After the solutions were discharged, the carrier resin was washed and dried to obtain the resin combined with a side-chain-protected peptide having the biotinylated N-terminus. Removal of the side-chain-protecting groups and cleavage of the peptide from the resin were carried out in the same manner as in Example 1 to obtain 47.3 mg of the crude peptide. The obtained crude peptide and 50 mg of DTT were dissolved in 1 ml of DMF, and the solution was allowed to stand at 50°C for one hour. The resulting solution was purified by HPLC using a reversed-phase column (Shiseido Co., Ltd., CAPCELL PAK C18 30 mm I.D. x 250 mm) in the same manner as in Example 1 to give 11.8 mg of a peptide having two free SH groups.
Mass spectrum [FAB-MS]: m/z=2510.8 (M+H⁺)

The obtained peptide was dissolved in 5 ml of a 2 M aqueous solution of acetic acid. After the resulting solution was diluted with water to 50 ml, dilute aqueous ammonia was added thereto to adjust the pH of the solution to 5.5. To the resulting mixture was added 0.5 ml of a 0.1 M aqueous solution of K₃Fe(CN)₆, followed by stirring at room temperature for 3 hours. After addition of 1 ml of acetic acid, the reaction mixture was purified by HPLC using a reversed-phase column (Shiseido Co., Ltd., CAPCELL PAK C18 30 mm I.D. x 250 mm) in the same manner as in Example 1 to give 3.8 mg of Compound 13.
Mass spectrum [FABMS]: m/z=2508.8 (M+H⁺)
Amino acid analysis: Glx 1.1 (1), Ser 1.0 (1), Arg 3.8 (4), Ala 1.1 (1), Val 0.8 (1), Leu 6.8 (7), Lys 2.0 (2), Cys 2.1 (2)

### Example 14 Synthesis of Compound 14 (SEQ ID NO: 14) (H-Leu-Ser-Thr-Cys-Lys-Thr-Ile-Asp-Met-Glu-Leu-Val-Lys-Arg-Lys-Arg-OH)

Condensation was carried out in the same manner as in Example 3 using 50 mg of a carrier resin (Wang resin) combined with 23 µmol of Fmoc-Arg(Pmc) as a starting material, and using Fmoc-Lys(Boc)-OH, Fmoc-Arg(Pmc)-OH, Fmoc-Lys(Boc)-OH, Fmoc-Val-OH, Fmoc-Leu-OH, Fmoc-Glu(OtBu)-OH, Fmoc-Met-OH, Fmoc-Asp(OtBu)-OH, Fmoc-Ile-OH, Fmoc-Thr(tBu)-OH, Fmoc-Lys(Boc)-OH, Fmoc-Cys(Trt)-OH, Fmoc-Thr(tBu)-OH, Fmoc-Ser(tBu)-OH and Fmoc-Leu-OH in turn. The condensation product was washed and dried to obtain the carrier resin to which a side-chain-protected peptide was bound. The reaction time for condensation with each amino acid was 60 minutes. Removal of the side-chain-protecting groups and cleavage of the peptide from the resin were carried out in the same manner as in Example 1 to obtain 59.8 mg of the crude peptide. The obtained crude peptide was purified by HPLC using a reversed-phase column (Shiseido Co., Ltd., CAPCELL PAK C18 30 mm I.D. x 250 mm) in the same manner as in Example 1 to give 7.7 mg of Compound 14.
Mass spectrum [FABMS]: m/z=1921.7 (M+H⁺)
Amino acid analysis: Asx 1.0 (1), Glx 1.1 (1), Ser 0.9 (1), Arg 2.2 (2), Thr 1.6 (2), Val 0.9 (1), Met 1.1 (1), Ile 1.0 (1), Leu 2.1 (2), Lys 3.1 (3), Cys 1.3 (1)

### Example 15 Synthesis of Compound 15 (SEQ ID NO: 15) (H-Leu-Lys-Leu-Lys-Val-Glu-Gln-His-Val-Glu-Leu-Tyr-Gln-Lys-Tyr-Ser-Asn-Asn-Ser-Trp-Arg-OH)

Condensation was carried out in the same manner as in Example 3 using 50 mg of a carrier resin (Wang resin) combined with 23 µmol of Fmoc-Arg(Pmc) as a starting material, and using Fmoc-Trp(Boc)-OH, Fmoc-Ser(tBu)-OH, Fmoc-Asn(Trt)-OH, Fmoc-Asn(Trt)-OH, Fmoc-Ser(tBu)-OH, Fmoc-Tyr(tBu)-OH, Fmoc-Lys(Boc)-OH, Fmoc-Gln(Trt)-OH, Fmoc-Tyr(tBu)-OH, Fmoc-Leu-OH, Fmoc-Glu(OtBu)-OH, Fmoc-Val-OH, Fmoc-His(Trt)-OH, Fmoc-Gln(Trt)-OH, Fmoc-Glu(OtBu)-OH, Fmoc-Val-OH, Fmoc-Lys(Boc)-OH, Fmoc-Leu-OH, Fmoc-Lys(Boc)-OH and Fmoc-Leu-OH in turn. The condensation product was washed and dried to obtain the carrier resin to which a side-chain-protected peptide was bound. The reaction time for condensation with each amino acid was 60 minutes. Removal of the side-chain-protecting groups and cleavage of the peptide from the resin were carried out in the same manner as in Example 1, except that a mixture of the same composition as in Example 1 additionally containing 5 mg/ml 2-methylindole was used and the standing time was 6 hours, to obtain 20.9 mg of the crude peptide. The obtained crude peptide was purified by HPLC using a reversed-phase column (Shiseido Co., Ltd., CAPCELL PAK C18 30 mm I.D. x 250 mm) in the same manner as in Example 1 to give 7.8 mg of Compound 15.
Mass spectrum [FABMS]: m/z=2663.5 (M+H⁺)
Amino acid analysis: Asx 2.0 (2), Glx 3.9 (4), Ser 2.0 (2), His 1.1 (1), Arg 1.1 (1), Tyr 2.2 (2), Val 1.8 (2), Leu 3.0 (3), Lys 3.0 (3)

### Example 16 Synthesis of Compound 16 (SEQ ID NO: 16) (Biotinyl-Gly-Arg-Arg-Leu-Lys-Leu-Lys-Val-Glu-Gln-His-Val-Glu-Leu-Tyr-Gln-Lys-Tyr-Ser-Asn-Asn-Ser-Trp-Arg-OH)

Condensation was carried out in the same manner as in Example 3 using 30 mg of a carrier resin (Wang resin) combined with 13.8 µmol of Fmoc-Arg(Pmc) as a starting material, and using Fmoc-Trp(Boc)-OH, Fmoc-Ser(tBu)-OH, Fmoc-Asn(Trt)-OH, Fmoc-Asn(Trt)-OH, Fmoc-Ser(tBu)-OH, Fmoc-Tyr(tBu)-OH, Fmoc-Lys(Boc)-OH, Fmoc-Gln(Trt)-OH, Fmoc-Tyr(tBu)-OH, Fmoc-Leu-OH, Fmoc-Glu(OtBu)-OH, Fmoc-Val-OH, Fmoc-His(Trt)-OH, Fmoc-Gln(Trt)-OH, Fmoc-Glu(OtBu)-OH, Fmoc-Val-OH, Fmoc-Lys(Boc)-OH, Fmoc-Leu-OH, Fmoc-Lys(Boc)-OH, Fmoc-Leu-OH, Fmoc-Arg(Pmc)-OH, Fmoc-Arg(Pmc)-OH and Fmoc-Gly-OH in turn. To the condensation product was added a solution which had been previously prepared by adding 562 µl of DMF containing 0.5 M HBTU and 0.5 M HOBt monohydrate and 562 µl of DMF containing 1 M DIEA to 61.9 mg of D-biotin (Nakalai Tesque, Inc.), and by stirring the mixture at room temperature for 5 minutes, and the resulting mixture was stirred for 4 hours. After the solution was discharged, the product was washed and dried to obtain the carrier resin to which a side-chain protected peptide having the biotinylated N-terminus was bound. In the condensation of an amino acid in step (d), HBTU was used in place of PyBOP and DIEA was used in place of NMM. Removal of the side-chain-protecting groups and cleavage or the peptide from the resin were carried out in the same manner as in Example 1, except that a mixture of the same composition as in Example 1 additionally containing 5 mg/ml 2-methylindole was used and the standing time was 6 hours, to obtain 44.2 mg of the crude peptide. The obtained crude peptide was purified by HPLC using a reversed-phase column (Shiseido Co., Ltd., CAPCELL PAK C18 30 mm I.D. 250 mm) in the same manner as in Example 1 to give 10.3 mg of Compound 16.
Mass spectrum [FABMS]: m/z=3260.0 (M+H⁺)
Amino acid analysis: Asx 2.1 (2), Glx 4.1 (4), Ser 2.0 (2), Gly 1.0 (1), His 1.0 (1), Arg 2.8 (3), Tyr 2.1 (2), Val 1.9 (2), Leu 3.0 (3), Lys 2.9 (3)

### Example 17 Conversion of Compound 10 into acetate

Compound 10 obtained according to the process of Example 10 (22.1 mg) was dissolved in 5 ml of a 1% aqueous solution of acetic acid, and the solution was passed through an anion exchange column (Asahi Chemical Industry Co., Ltd., Asahi Pack ES-502N 21.5 mm I.D. x 100 mm) to remove TFA and convert the compound into acetate. Elution of the peptide was carried out with a 1% aqueous solution of acetic acid, followed by detection at 220 nm. The eluate was lyophilized to give 20.0 mg of acetate of Compound 10.

### Example 18 Conversion of Compound 12 into acetate

Compound 12 obtained according to the process of Example 12 (17.5 mg) was dissolved in 5 ml of a 1% aqueous solution of acetic acid, and the solution was passed through an anion exchange column (Asahi Chemical Industry Co., Ltd., Asahi Pack ES-502N 21.5 mm I.D. x 100 mm) to remove TFA and convert the compound into acetate. Elution of the peptide was carried out with a 1% aqueous solution of acetic acid, followed by detection at 220 nm. The eluate was lyophilized to give 13.0 mg of acetate of Compound 12.

The biological activities of Compounds (I) and the screening methods are described by the following Examples.

### Example 19 Activity to release active TGF-β from latent TGF-β examined by measuring the degree of binding of TGF-β to bovine vascular smooth muscle cells, porcine vascular smooth muscle cells, bovine capillary endothelial cells or bovine aortic endothelial cells

### (1-1) ¹²⁵I-Labeling of LLTGF-β

LLTGF-β isolated and purified from human platelets by the method of Okada, et al. [Journal of Biochemistry, 106, 304 (1989)] was ¹²⁵I-labeled according to the chloramine T method [Molecular & Cellular Biology, 2, 599 (1982)] in the following manner.

To 2 µg of LLTGF-β were added 25 µl of 1 M K-phosphate buffer (pH 7.5), 37 MBq of Na-¹²⁵I (DuPont NEN) and 10 µl of chloramine-T (0.5 mg/100 µl in 0.05 M K-phos. buffer, Wako Pure Chemical Industries, Ltd.), followed by stirring at room temperature for 40 seconds. After 15 µl of Na₂S₂O₅ (1 mg/100 µl in 0.05 M K-phos. buffer) was added, the mixture was stirred at room temperature for 5 seconds, followed by addition of 100 µl of an aqueous solution of tyrosine (1 mg/ml). The reaction mixture was applied to a Sephadex G25 column and the eluate was taken in 500 µl fractions. Each fraction was subjected to measurement of radioactivity using a γ -counter (ARC-2000, Aloka Co., Ltd.). The fractions containing labeled protein were selected for use in the experiment.

### (1-2) Preparation of cells

The cells to be used in the experiment were obtained according to the method of Sato, et al. [Journal of Cell Biology, 123, 1249 (1993)]. Bovine vascular smooth muscle cells (BSMC) and porcine vascular smooth muscle cells (PSMC) were isolated from bovine aorta and porcine aorta, respectively, and cultured by the explant method [Journal of Cell Biology, 50, 172 (1971)]. Bovine capillary endothelial cells (BCEC) were isolated from bovine adrenal capillary and cultured. Bovine aortic endothelial cells (BAEC) were isolated from bovine aortic and cultured.

### (1-3) Measurement of the degree of TGF-β binding

The following procedure was carried out according to the method of Sato, et al. [Journal of Cell Biology, 123, 1249 (1993)].

BSMC, PSMC and BCEC isolated and cultured in the above-described manner were respectively put into 35 mm dishes in an amount of 4 x 10⁴ cells/dish. On the next day, the medium was replaced by Dulbecco's Modified Eagle's Medium (DMEM, Nissui Pharmaceutical Co.) containing 0.1% bovine serum albumin (BSA), followed by incubation for 5 hours. After the cells were washed with 2 ml of phosphate-buffered saline containing 0.1 g/l each of MgCl₂ · 6H₂O and CaCl₂ [hereinafter sometimes referred to as PBS(+); phosphate-buffered saline containing neither MgCl₂ · 6H₂O nor CaCl₂ is referred to as PBS(-)], the medium was replaced by 1 ml of ice-cold DMEM containing 0.1% BSA, 2 ng/ml ¹²⁵I-LLTGF-β and a test compound, followed by incubation at 4°C for 3 hours. The test compound was added as a solution in dimethyl sulfoxide (DMSO) to give a final DMSO concentration in the system of 0.1%. After being washed three times with 2 ml of ice-cold PBS(+), the cells were lysed with 900 µl of 0.5% Triton X-100 (room temperature, 0.5-1 hour), and 800 µl of the lysate was subjected to measurement of radioactivity using a γ-counter.

The results are shown in Figs. 1 and 2 and Tables 3 to 6.

**Table 3**

| Test group | Radioactivity (cpm), n=4 |
|---|---|
| Vehicle | 163.1 ± 28.3 |
| Cell free | 70.6 ± 10.2 |
| Compound 12 | 2739.9 ± 182.8 |

Table 3 shows the degree of binding of active TGF-β to bovine capillary endothelial cells (BCEC) as determined by measuring the radioactivity. "Vehicle" lane shows the radioactivity when a solution without a test compound was added, "cell free" lane shows the radioactivity when ¹²⁵I-LLTGF-β alone was added to a plate without a cell, and the other lane shows the radioactivity when the test compound was added at a concentration or 30 µg/ml.

**Table 4**

| Test group | Radioactivity (cpm), n=4 |
|---|---|
| Vehicle | 416.8 ± 89.6 |
| Cell free | 70.6 ± 10.2 |
| Compound 14 | 3369.9 ± 166.8 |
| Compound 15 | 5621.1 ± 889.2 |

Table 4 shows the degree of binding of active TGF-β to bovine capillary endothelial cells (BCEC) as determined by measuring the radioactivity. "Vehicle" lane shows the radioactivity when a solution without a test compound was added, "cell free" lane shows the radioactivity when ¹²⁵I-LLTGF-β alone was added to a plate without a cell, and the other lanes show the radioactivity when the respective test compound was added at a concentration of 100 µg/ml.

**Table 5**

| Test group | Radioactivity (cpm), n=4 |
|---|---|
| Vehicle | 1076.5 ± 12.0 |
| Compound 13 | 4704.0 ± 939.9 |

Table 5 shows the decree of binding of active TGF-β to bovine aortic endothelial cells (BAEC) as determined by measuring the radioactivity. "Vehicle" lane shows the radioactivity when a solution without a test compound was added, and the other lane shows the radioactivity when, the test compound was added at a concentration of 100 µg/ml.

**Table 6**

| Test group | Radioactivity (cpm), n=4 |
|---|---|
| Vehicle | 792.0 ± 107.0 |
| Compound 16 | 10495.0 ± 803.0 |

Table 6 shows the degree or binding of active TGF-β to bovine aortic endothelial cells (BAEC) as determined by measuring the radioactivity. "Vehicle" lane shows the radioactivity when a solution without a test compound was added, and the other lane shows the radioactivity when the test compound was added at a concentration of 100 µg/ml.

As shown in Figs. 1 and 2 and Tables 3 to 6, the degree of binding of TGF-β to cells was increased and the release of active TGF-β from latent TGF-β was promoted by Compounds 1 to 3, 5 to 9 and 13 to 16 at a concentration of 100 µg/ml and Compound 12 at a concentration or 30 µg/ml.

Compounds promoting the release of active TGF-β from latent TGF-β and increasing the degree of binding of TGF-β to cells can be obtainable by the method of the present invention.

### Example 20 Activity to release active TGF-β from latent TGF-β examined by measuring the degree of migration of bovine vascular endothelial cells

The following procedure was carried out according to the method of Sato, et al. [Journal or Cell Biology, 123, 1249 (1993)].

BCEC were cultured in a 35 mm dish to make a confluent layer and then the medium was replaced by DMEM containing 0.1% BSA, followed by incubation for 5 hours. After a part of the cells were scraped with a razor, the remaining cells were washed with PBS(-), followed by incubation in DMEM containing 0.1% BSA and a test compound. After 24 hours, the number of the cells which migrated in four fileds of a microscope (IX70, Olympus) was counted or each dish. The test compound was added in the same manner as in Example 19. The results are shown in Figs. 3 to 5. The migration of cells was inhibited and the release of active TGF-β from latent TGF-β was promoted by Compounds 3 and 5 to 9 at a concentration of 50 µg/ml. The migration of cells was also inhibited by Compound 4 at a concentration of 100 µg/ml, and Compound 10 showed the migration inhibitory effect even at 10 µg/ml.

Compounds promoting the release of active TGF-β from latent TGF-β and increasing the degree of binding of TGF-β to cells can be screened by the method of the present invention.

### Example 21 Determination of active TGF-β by luciferase assay

The amount of active TGF-β was determined by measuring the luminescence of mink lung epithelial cells (MLEC) carrying the luciferase gene introduced downstream of the PAI-1 promoter by luciferase assay system (Promega) according to the method of Abe, et al. [Analytical Biochemistry, 216, 276 (1994)] as described below.

BCEC were cultured in a 24-well plate to make a confluent layer and then the medium was replaced by DMEM containing 0.1% BSA. After 6 hours, the cells were scraped reticulately with a comb and the remaining cells were washed with PBS(-), followed by incubation in DMEM containing 0.1% BSA and a test compound for 24 hours. The resulting culture supernatant was taken as a sample. Six hours before the sampling of this culture supernatant, the above-mentioned MLEC were put into wells of a 96-well plate in an amount of 2.8 x 10⁴ cells/well. After the cells were cultured in DMEM containing 10% fetal calf serum (FCS) for 6 hours, the medium was replaced by the above culture supernatant sample, followed by incubation for 16 hours. The cells were washed twice with PBS(-) and then subjected to measurement of active TGF-β concentration by lucuferase assay system. The results are shown in Fig. 6. Active TGF-β was released by the addition of Compounds 3 and 10. Particularly, Compound 10 showed a remarkable effect even at a concentration of 10 µg/ml.

Compounds promoting the release of active TGF-β from latent TGF-β and increasing the degree of binding of TGF-β to cells can be screened by the method of the present invention.

### Industrial Applicability

The present invention provides novel peptides having an activity to promote the activation of latent TGF-β by enhancing the binding of latent TGF-β to a cell membrane, and pharmaceutically acceptable salts thereof.

According to the methods of screening compounds of the present invention, compounds having an activity to promote the release of active TGF-β from latent TGF-β or an activity to promote the binding of latent TGF-β to a cell membrane can be selected.

The compounds obtained by the screening methods of the present invention, Compounds (I) and pharmaceutically acceptable salts thereof have inhibiting activity or promoting activity on the binding of latent TGF-β to cells or on the conversion of latent TGF-β into TGF-β. Thus, they are useful for the treatment or prevention of diseases such as cancer, diabetic retinopathy, atherosclerosis, bone fracture, myocardial infarction, myocardial disorder after ischemia reperfusion, cerebral infarction, retinal detachment, glomerulonephritis, diabetic nephropathy, renal graft rejection, HIV nephropathy, sudden pulmonary fibrosis, autoimmune pulmonary fibrosis, hepatic cirrhosis, venous constrictive hepatopathy (often occurring after treatments of cancer), systemic sclerosis, keloid, eosinophilia-muscle ache syndrome, re-stricture after angioplasty, intraocular fibrosis, rheumatic arthritis and nasal polyp.

## Claims

1. A peptide having an activity to promote the release of active TGF-β from latent TGF-β or an activity to promote the binding of latent TGF-β to a cell membrane, or a pharmaceutically acceptable salt thereof.

2. A peptide or a pharmaceutically acceptable salt thereof according to Claim 1, wherein said peptide is represented by general formula (I):
R¹-A-R² (I)
(wherein R¹ represents hydrogen, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; R² represents hydroxy, substituted or unsubstituted alkoxy, or substituted or unsubstituted amino; and A represents an amino acid sequence which is selected from partial sequences of a TGF-β precursor sequence and in which 1 to 5 amino acid residues may be deleted, substituted or added; and at two amino acid residues selected from the amino acid residues including the N-terminal and C-terminal amino acid residues in the sequence, the N-terminal amino group or a side-chain amino group and the C-terminal carboxyl group or a side-chain carboxyl group may form an amide bond represented by CO-NH or a reversed amide bond represented by NH-CO, or side-chain thiol groups may form a disulfide bond).

3. A peptide or a pharmaceutically acceptable salt thereof according to Claim 2, wherein A is an amino acid sequence selected from partial sequences of an amino acid sequence selected from the sequences of amino acids 30 to 60, 142 to 186, and 269 to 297 in the human TGF-β 1 precursor sequence and the sequences of TGF-β precursors other than human TGF-β 1 corresponding to the sequences of amino acids 30 to 60, 142 to 186, and 269 to 297 in the human TGF-β 1 precursor sequence when aligned with the human TGF-β 1 sequence, and 1 to 5 amino acid residues in said partial sequence may be deleted, substituted or added.

4. A peptide or a pharmaceutically acceptable salt thereof according to Claim 2, wherein A is an amino acid sequence selected from partial sequences of an amino acid sequence selected from the sequences of amino acids 30 to 60, 142 to 186, and 269 to 297 in the human TGF-β 1 precursor sequence and the sequences of TGF-β precursors other than human TGF-β 1 corresponding to the sequences of amino acids 30 to 60, 142 to 186, and 269 to 297 in the human TGF-β 1 precursor sequence when aligned with the human TGF-β 1 sequence.

5. A peptide or a pharmaceutically acceptable salt thereof according to Claim 2, wherein A is an amino acid sequence selected from the sequences of SEQ ID NOS: 1 to 16 in which 1 to 5 amino acid residues may be deleted, substituted or added.

6. A peptide or a pharmaceutically acceptable salt thereof according to Claim 2, wherein A is an amino acid sequence selected from the sequences of SEQ ID NOS: 1 to 16.

7. A method of screening a compound to be used for the treatment or prevention of TGF-β-related diseases, which comprises:
measuring the amount of latent TGF-β bound to animal cells after addition of latent TGF-β to said cells;
measuring the amount of latent TGF-β bound to animal cells after addition of latent TGF-β and a compound to be evaluated to said cells; and
evaluating the inhibiting activity or promoting activity of said compound on the binding of latent TGF-β to animal cells from the change in the amount of latent TGF-β bound to animal cells caused by the addition of said compound.

8. A method according to Claim 7, wherein said animal cells are vascular endothelial cells.

9. A method according to Claim 7 or 8, wherein the promoting activity of said compound on the binding of latent TGF-β to animal cells is evaluated.

10. A method of screening a compound to be used for the treatment or prevention of TGF-β-related diseases, which comprises:
measuring the amount of TGF-β after addition of a peptide or a pharmaceutically acceptable salt thereof according to any of Claims 1-6 to animal cells;
measuring the amount of TGF-β after addition of a compound to be evaluated and a peptide or a pharmaceutically acceptable salt thereof according to any of Claims 1-6 to animal cells; and evaluating the inhibiting activity or promoting activity of said compound on the conversion of latent TGF-β into TGF-β from the change in the amount of TGF-β caused by the addition of said compound.

11. A method according to Claim 10, wherein said animal cells are vascular endothelial cells.

12. A method according to Claim 10 or 11, wherein the inhibiting activity of said compound on the conversion of latent TGF-β into TGF-β is evaluated.

13. A compound to be used for the treatment or prevention of TGF-β-related diseases, which is obtainable by a method according to any of Claims 7-12, or a pharmaceutically acceptable salt thereof.
